# EUROPEAN PATENT APPLICATION

(11) **EP 4 361 972 A1**
(43) Date of publication of application: **01.05.2024**
(21) Application number: 22204778.9
(22) Date of filing: 31.10.2022
(51) Int. Cl.: G06V 40/10, A61B 5/1455, A61B 5/00, A61B 5/024, G06V 10/26, G06V 10/56, G06V 40/16

(54) **PHOTOPLETHYSMOGRAPHY SIGNAL EXTRACTION**

(71) Applicant: Koninklijke Philips N.V., 5656 AG Eindhoven (NL)
(72) Inventor: DEN BRINKER, Albertus Cornelis, 5656AG Eindhoven (NL); WUELBERN, Jan Hendrik, 5656AG Eindhoven (NL); WEISS, Steffen, 5656AG Eindhoven (NL); PAPPOUS, Ioannis, 5656AG Eindhoven (NL)
(74) Representative: Philips Intellectual Property & Standards

(57) **Abstract**

A device (10) and method are disclosed for use in camera-based photoplethysmography. The device comprises an input (11) for camera images and a subset generator (12) to divide an image region where a tissue of interest is presumed to be present into a first plurality of pixel subsets, as well as a second, presumed irrelevant, region into a second plurality of subsets. A signal dynamics analyzer (13) determines a dynamic characteristic per subset. A clustering processor (14) clusters, respectively, the first and second plurality of subsets based on their dynamic characteristics. A selector (15) selects (a) cluster(s) from the first plurality of subsets based on size and/or morphology, thereby excluding each cluster that substantially corresponds in its associated signal dynamics to a cluster of the second plurality of subsets. A region-of-interest defining the selected cluster(s), and/or a PPG signal extracted therefrom, is provided as output (16).

## Description

### FIELD OF THE INVENTION

The present invention relates to the field of image processing, and particularly for determining a photoplethysmography (PPG) signal from a subject based on camera images. More specifically, the invention relates to a device, method, system and computer program product for use in determining a photoplethysmography signal by means of camera observation.

### BACKGROUND OF THE INVENTION

Photoplethysmography (PPG) is a technique that uses changes in light interaction, e.g. light absorption, to measure blood volume changes in the microvasculature. For example, a simple PPG sensor may consist of a light source to illuminate living tissue, such as the skin, mucous membranes, and other translucent tissues, and a light detector, such as a photodiode, phototransistor and/or photomultiplier tube, to measure the amount of light that is scattered and/or absorbed by the tissue. The detected signal is indicative of blood volume changes in the microvasculature, and thus provides a noninvasive way to measure blood volume changes in (near) real-time. PPG has a wide range of applications, including monitoring of blood oxygenation, pulse rate, current cardiac phase, and blood pressure. The information contained in the PPG signal can for example be used in triggering and/or gating during magnetic resonance imaging (MRI) procedures.

Furthermore, it is known in the art to use camera observation of a subject, e.g. a patient undergoing a medical imaging or treatment procedure, to obtain useful signals indicative of physiological parameters of the subject. For example, knowledge of the subject's cardiovascular and/or respiratory function may be useful to monitor the patient's state, e.g. health, in a procedure, to take into account in processing images obtained in a diagnostic imaging procedure and/or to guide or control the image acquisition of the performed procedure in a relevant manner.

Remote photoplethysmography (rPPG), also referred to as camera-PPG or video-PPG, is a method of non-contact PPG signal extraction that uses a camera to capture images of the tissue under investigation. Camera-based PPG has several advantages over conventional (contact) PPG, including the ability to capture a larger area of the tissue, which can be helpful in cases where the tissue is not evenly illuminated, and/or the ability to capture multiple PPG signals from different parts of the tissue simultaneously, e.g. to improve the accuracy of the result by averaging.

In a typical approach to obtain an rPPG signal, a region of interest (ROI), e.g. a patch of skin tissue, is first identified in one or more images (e.g. a video stream) obtained by the camera. From dynamic changes in the pixel values corresponding to this ROI, the PPG is then constructed. This may include various image/signal processing techniques, such as downmixing of color signals and post-processing to clean the signal from undesired components. From the PPG signal thus obtained, features of interest can then be extracted, such as the subject's pulse rate and/or markers (e.g. points in time) that identify one or more specific stages in the cardiac cycle. Such markers may for example be used by a trigger generator system to trigger an MRI pre-pulse and/or to control the data acquisition windows.

In MRI, the ROI identification may advantageously use prior knowledge about the MRI environment, since the environment typically remains substantially the same over different sessions. Illumination level information, e.g. favouring a region that is neither too dark nor too bright in order to select a well-illuminated patch of skin, and pulsatility information (i.e. indicative of living skin) are also typically taken into account.

The dynamic range of the camera-based PPG signal itself is very small, i.e. in the order of 10⁻³ relative to the mean illumination (mean pixel value offsets) and also small to other factors that could influence the detected pixel signals, such that carefully controlled illumination conditions may generally be an important consideration to achieve continuous and accurate monitoring. Therefore, the use of a high-quality, stable infrared illumination source may be considered advantageous. A simple set-up may use a single-channel near infrared (NIR) illumination source and a camera picking up the NIR signals, after modulation by interaction with the tissue of interest. The signals of the different pixels in the ROI area can be combined to construct the PPG signal.

Nuisance components in the signals can be eliminated by signal decomposition techniques. This may include disturbances due to motion of the subject, i.e. motion signals in the image can be measured and eliminated from the (raw) PPG signal. Similarly, generic light variations can be measured and eliminated from the PPG signal.

An illustrative approach of taking known pulsatility properties of a skin-based PPG signal may be commonly referred to as a "living skin model." In such approach, the camera image, or a part thereof that is preselected based on prior knowledge (such as a region in the image in which a patient is typically positioned for a predetermined procedure), is divided into small patches, e.g. a grid of rectangular patches abutting onto their neighbours to form a partition of the image or preselected part thereof.

A mean pixel intensity level and spread (e.g. a standard deviation) can then be determined for each patch, as well as motion vectors for each patch (e.g. using an optical flow algorithm). When the average pixel level, the spread and the motion all satisfy predetermined conditions, the patch is selected as a homogeneous non-moving patch for further use, e.g. thus pruning the patches that do not show these desirable properties. For example, the average pixel level condition may correspond to lighting conditions that fall with a comfortable margin in the dynamic range of the camera system and that are consistent with the typical characteristics of the tissue of interest under such lighting conditions. When the spread and motion are below (respective) predetermined thresholds some level of stability to determine the signal of interest from can be assumed, e.g. the patch would not be excessively affected by various sources of noise and/or errors, nor by strong motion.

For the patches that are not withheld by the pruning process, the average value as function of time can then be analyzed. After applying a Fourier transform, frequency components that lie within a predetermined range, e.g. representative of the heart rate band, can be examined to find spectral peaks inside this range. If the strongest peak is sufficiently distinct, e.g. the energy at the peak relative to the average of the range is sufficiently high (and/or relative to the second strongest peak), the patch is associated with the frequency of this primary peak (the strongest spectral peak). This value can be referred to as the patch frequency. Finally, by clustering the patch frequencies, the largest cluster can be selected as the most likely candidate for being representative of the PPG signal, i.e. the pixels belonging to the patches in this cluster can be used to define the region of interest to extract the PPG signal after this initial setup/calibration procedure.

For example, US 10441173 B2 describes a prior-art method for extracting physiological information, e.g. a PPG signal, from remote camera observation data. A data stream including image data is received that represents an observed region of the subject of interest. A plurality of sub-regions in this region are defined, and a classifier classifies the sub-regions into an indicative type and an auxiliary type of region, in which the former includes (a) region(s) that at least partially represent the subject of interest, and the latter includes (a) reference region(s). The sub-region(s) classified as region of interest are then further processed to obtain the vital information, e.g. the PPG signal, of interest.

### SUMMARY OF THE INVENTION

It is an object of embodiments of the present invention to provide good and efficient means and methods for use in determining a PPG signal from a subject, e.g. a patient undergoing a medical imaging procedure, based on remote observation, e.g. from (a video stream of) camera images of the subject. Particularly, a region-of-interest is determined that is particularly suitable to extract such PPG signal from in the camera images, and/or this ROI is used to extract and provide such PPG signal.

It is an advantage of embodiments of the invention that remote imaging may be used, e.g. such that direct contact with the subject is not necessary and/or such that equipment can be placed at a substantial distance from the subject. For example, in a medical environment, it may be preferable to keep the direct vicinity of the patient clear of obstructions, e.g. such that a procedure can be performed without unnecessary hindrance.

Furthermore, in medical imaging, the diagnostic imaging technology may interfere with other electronics.

In magnetic resonance imaging (MRI), particularly, the hardware of the MR scanner, e.g. radiofrequency antennae and/or magnetic field gradient coils could interfere with a correct operation of further electronic devices, e.g. the PPG sensor could pick up noise due to radiofrequency signals and/or be affected by strong magnetic fields (and/or field gradients and/or dynamic field changes). Similarly, the illumination sources used to illuminate the skin of the patient for PPG detection may be disturbed by the MR scanner hardware giving rise to illumination changes that may disturb PPG detection.
It is furthermore an advantage that an accurate and/or stable PPG signal can be obtained, e.g. by reducing and/or avoiding carrying over nuisance signals, e.g. random noise or systematic errors, to the PPG output signal being inferred from the raw data stream.

Therefore, the output signal thus provided may require less post-processing, e.g. further cleaning of the signal. This may improve the efficient use of computational resources, e.g. processing power, memory resources, power consumption and the like, and/or may improve the accuracy and stability of results derived from the output. For example, many signal cleaning strategies require that the measured disturbance signal and the disturbance in the raw PPG are synchronous, a condition which is not always satisfied. By careful selection of pixels from which the signal is derived, a more stable and representative PPG signal can be obtained, in which residual nuisance components may be generally (relatively) smaller and less random.

It is an advantage of embodiments of the present invention that a useful signal can be determined automatically (e.g. algorithmically) from camera observation of a subject. In many environments, a suitable camera system may already be present, or can be installed in an easy upgrade, such that embodiments may be applied to an existing system without complex and/or costly interventions.

It is an advantage of embodiments of the present invention that a signal can be determined from camera observation that can be used for triggering and/or system control in a diagnostic imaging procedure using a scanner system, such as an MRI, CT, SPECT or PET scanner, which may advantagesouly improve the diagnostic image quality. Such trigger signal may also be used in, for example, radiotherapy and similar (e.g. therapeutic) procedures, e.g. are not necessarily strictly limited to medical imaging applications. Furthermore, other areas of application for PPG acquisition are not necessarily excluded either.

It is an advantage of embodiments of the present invention that a good region-of-interest (ROI) selection of pixels representative of a PPG pulsative signal, having such substantial component, can be obtained. Particularly, a-priori knowledge may be advantageously used to prevent undesirable signal components, i.e. nuisance signals, noise and/or other disturbances, from contributing to the extracted PPG signal. For example, a refined pixel screening process may be applied to select a suitable ROI, based on knowledge of the properties of desirable vs. undesirable areas and/or pixel signal content. Knowledge of non-skin locations (or, more generally, locations not representative of the tissue type of interest to monitor) can be advantageously used to extract pulsatile features. Furthermore, morphological knowledge of the characteristics of promising candidate areas can be applied in the ROI selection. Prior knowledge of the properties of the pulsatility associated with PPG may also be advantageously used.

A device, system, method and computer program product in accordance with embodiments of the present invention achieves the above objective.

In a first aspect, the present invention relates to a method, e.g. a computer-implemented method, for use in extracting a photoplethysmography signal indicative of a subject's physiology based on remote camera observation (e.g. from a video stream obtained by said remote camera observation), e.g. a method for determining the photoplethysmography signal and/or for determining an image region-of-interest for use in extracting the photoplethysmography signal. The method comprises acquiring camera images (e.g. a stream of video images) from a camera configured to monitor at least one body part of the subject. The method comprises dividing a first region of the camera images, representative of an image part where a tissue of interest is presumed to be present, into a first plurality of subsets of pixels and dividing a second region of the camera images, different from the first region and representative of an image part where the tissue of interest is presumed to be absent, into a second plurality of subsets of pixels.

The method furthermore comprises determining, for each subset of the (e.g. pruned) first and second plurality of subsets, at least one dynamic characteristic value that is indicative of temporal signal dynamics of (the pixels forming) the subset in a sequence of the camera images.

The method comprises clustering, respectively, the (e.g. pruned) first plurality of subsets and the (e.g. pruned) second plurality of subsets, based on the at least one dynamic characteristic value, so as to group subsets of pixels into clusters of similar signal dynamics. By using (substantially) the same clustering variable (e.g. a peak frequency and/or magnitude, or a value or values substantially equivalent thereto) for clustering the subsets (e.g. patches) of the first region as well as clustering the subsets (e.g. patches) of the second region, information from presumed non-relevant clusters in the second region can be taken into account in selecting one or more relevant clusters in the first region, as discussed hereinbelow.

The method comprises selecting at least one cluster from the clusters of subsets obtained by the step of clustering the first plurality of subsets based on cluster size and/or cluster morphology, in which the step of selecting the cluster(s) excludes from said selection each cluster of the first plurality of subsets that substantially corresponds in its associated temporal signal dynamics to the temporal signal dynamics of a cluster of the second plurality of subsets.

The method further comprises providing an output. The output comprises a region-of-interest definition to define the pixels forming the selected at least one cluster and/or a photoplethysmography signal extracted from the region-of-interest in the camera images, e.g. based on a further sequence of images acquired by the camera.

In a method in accordance with embodiments of the present invention, the first region, respectively the second region, may be divided into said first, respectively second, plurality of pixel subsets based on predetermined prior-knowledge assumptions of where the tissue of interest is likely present, respectively absent, in the camera images, given a known setup of the camera with respect to a volume in space where the subject is to be positioned.

In a method in accordance with embodiments of the present invention, selecting the at least one cluster may comprise selecting the largest cluster or clusters from the first plurality of subsets that is, or are, not excluded based on said correspondence to a cluster of the second plurality of subsets.

In a method in accordance with embodiments of the present invention, selecting the at least one cluster may comprise determining for each cluster of the first plurality of subsets a shape measure representative of a shape of the image area formed by the pixels (or patches) in the cluster. The selection may be based on the shape measure of the at least one selected cluster being consistent with a predetermined target shape of an area of the tissue of interest, such as an oval or ellipse shape indicative of the exposed skin of the face or of the forehead of the subject.

In a method in accordance with embodiments of the present invention, selecting the at least one cluster may be based on a ranking of the clusters of the first plurality of subsets (e.g. that are not excluded based on said correspondence to a cluster of the second plurality of subsets), in which said ranking combines a score based on a size of the cluster and a score based on the shape measure. The ranking may, optionally, also include a further score based on the correspondence of the cluster with (e.g. the best match of) clusters of the second plurality of subsets.

In a method in accordance with embodiments of the present invention, the first region, respectively the second region, may be divided into said first, respectively second, plurality of pixel subsets by forming a partition of the first region, respectively the second region, into blocks or patches based on a spatial distance metric and/or into subsets based on an image codomain distance and/or based on a combination thereof, such that each subset groups pixels that are close together in space and/or in value. For example, in embodiments where different pixel subset sizes are considered (e.g. in an optimization strategy as discussed hereinbelow), the grouping of pixels into the (first, resp. second) subsets may switch, or shift in weight, from forming subsets that are more or only based on distance in the spatial domain to forming subsets that are more or only based on distance in the image codomain (e.g. the pixel value domain).

In a method in accordance with embodiments of the present invention, dividing the first region, respectively the second region, into said first, respectively second, plurality of pixel subsets may comprise an image segmentation and/or image processing of an image acquired by the camera and/or of at least one image and/or spatial information acquired by a further camera and/or a further spatial information source (e.g. a 3D camera, a diagnostic imaging apparatus, ...) to determine said first and second regions.

In a method in accordance with embodiments of the present invention, dividing the first region, respectively the second region, into said first, respectively second, plurality of pixel subsets may comprise an image subtraction between an image acquired with the subject present and a blank image acquired without the subject present, and/or a similar image background compensation technique, so as to detect the first region and/or the second region.

A method in accordance with embodiments of the present invention may comprise at least performing the step of dividing the first region into said first plurality of pixel subsets repeatedly (e.g. to apply an optimization strategy), such that different iterations correspond to different values of at least one optimization parameter representative of at least the size of the pixel subsets. The method may further comprise evaluating a quality metric based on the pixel subsets obtained in each iteration so as to select the parameter value for which a sufficient or optimal value of the quality metric Is obtained, and using the plurality of pixel subsets obtained for said selected parameter value in the further steps of the method, e.g. such that the clustering, and therefore also the region-of-interest determined therefrom, is based on subsets corresponding to said parameter choice.

In a method in accordance with embodiments of the present invention, the at least one optimization parameter may further comprise a size of a temporal observation window used for determining the at least one dynamic characteristic value per subset.

In a method in accordance with embodiments of the present invention, the quality metric used in said optimization strategy may comprise or consist of a signal-to-noise ratio.

In a method in accordance with embodiments of the present invention, determining the at least one dynamic characteristic value may comprise performing a Fourier transform in the temporal domain to obtain one or more temporal frequency characteristics.

A method in accordance with embodiments of the present invention may comprise pruning the first plurality of subsets based on predetermined criteria for one or more values determined on a subset-per-subset basis, so as to reject subsets that have a low likelihood of corresponding to homogeneous areas of the tissue of interest, and may comprise pruning the second plurality of subsets, as well, based on (the same and/or similar) predetermined criteria. Thus, the clustering may be applied to the pruned first plurality of subsets and to the pruned second plurality of subsets.

In a method in accordance with embodiments of the present invention, said pruning may be performed based on said predetermined criteria, in which the predetermined criteria may comprise one or more criteria for a mean pixel value per subset and/or a spread of the pixel values per subset and/or for at least one value indicative of motion associated with the subset, such as to reject subsets that show excessive motion, an inhomogeneous pixel value distribution and/or a pixel value that is, on average, outside a predetermined target range. It will be understood that various alternative measures for the mean (e.g., generally, a measure of centrality of the pixels' values over the subset), the spread (e.g., generally, a measure of dispersion of the pixels' values over the subset) and/or motion (e.g. a measure that correlates with or is associated with movement, e.g. such as determined by optical flow) may be used.

A method in accordance with embodiments of the present invention may comprise a further pruning of the first and/or second plurality of subsets (e.g. which may have already been pruned a first time by the step described hereinabove) based on said at least one dynamic characteristic value, before applying said clustering to the further pruned first and/or second plurality of subsets. The further pruning may be based on a predetermined criterion (or criteria) for the spectral energy of the largest spectral peak of the Fourier spectrum determined for each subset and/or based on a value determined therefrom.

In a second aspect, the present invention relates to a device for use in extracting a photoplethysmography signal indicative of a subject's physiology based on remote camera observation, e.g. a device for extracting the photoplethysmography signal and/or for providing a region-of-interest definition to be used to extract the photoplethysmography signal. The device comprises an input for receiving camera images from a camera configured to monitor at least one body part of the subject.

The device comprises a subset generator for dividing a first region, representative of an image part in said camera images where a tissue of interest is presumed to be present, into a first plurality of subsets of pixels and dividing a second region, different from the first region and representative of an image part in said camera images where the tissue of interest is presumed to be absent, into a second plurality of subsets of pixels.

The device comprises a signal dynamics analyzer for determining, per subset of the first plurality of subsets and (e.g. independently) per subset of the second plurality of subsets, at least one dynamic characteristic value that is indicative of temporal signal dynamics of the subset in a sequence of the camera images received via the input.

The device comprises a clustering processor for clustering, respectively, the first plurality of subsets and the second plurality of subsets (e.g. clustering the first plurality of subsets independently from clustering the second plurality of subsets, and vice versa), based on the at least one dynamic characteristic value, so as to group subsets of pixels into clusters with similar signal dynamics. It will be understood that a (image) clustering algorithm may be generally considered to take spatial relations into account as well, e.g. to group subsets together that are both close in space as similar in the associated dynamic characteristic(s).

The device comprises a selector for selecting at least one cluster from the clusters of the first plurality of subsets provided by the clustering processor based on cluster size and/or cluster morphology. The selector is adapted to exclude from said selection each cluster of the first plurality of subsets that substantially corresponds in its associated signal dynamics to the signal dynamics of a cluster of the second plurality of subsets.

The device comprises an output to output a region-of-interest definition that defines the pixels forming the at least one cluster selected by the selector for use in extracting a photoplethysmography signal from camera images acquired by the camera and/or to output a photoplethysmography signal extracted from the region-of-interest in camera images received via the input.

A device in accordance with embodiments of the present invention may comprise said camera, e.g. a camera suitable for PPG signal extraction, such as an infrared camera, color camera and/or camera with predetermined PPG filter, e.g. a selective green filter. It will be understood that the device may also comprise (optionally) a light source to illuminate the subject (or at least the body part of interest), e.g. with light comprising a suitable spectral component corresponding to the sensitivity of the camera. The camera may comprise one camera, or a combination of multiple cameras.

A device in accordance with embodiments of the present invention may comprise a photoplethysmography signal extractor to extract, from the region-of-interest in (a sequence of) said camera images, the photoplethysmography signal to be provided as output.

In a device in accordance with embodiments of the present invention, the subset generator may be adapted to divide the first region, respectively the second region, into the first, respectively the second, plurality of pixel subsets based on predetermined prior-knowledge assumptions of where the tissue of interest is likely present, respectively absent, given a known setup of the camera with respect to a volume in space where the subject is to be positioned. For example, the regions may be defined via a user interface, may be preconfigured (e.g. stored in a configuration memory and/or hardcoded), or may be retrieved from an external data storage, e.g. where it was stored during a calibration procedure of the camera setup.

In a device in accordance with embodiments of the present invention, the selector for selecting the at least one cluster may be adapted to select the largest cluster(s) from the first plurality of subsets that is (or are) not excluded based on said correspondence to a cluster of the second plurality of subsets.

In a device in accordance with embodiments of the present invention, the selector may be adapted to determine, for each cluster of the first plurality of subsets, a shape measure representative of the shape of that cluster, and for selecting said at least one cluster based on the shape measure of the selected cluster being consistent with a predetermined target shape of an area of the tissue of interest.

In a device in accordance with embodiments of the present invention, the selector may be adapted to determine a ranking of the clusters of the first plurality of subsets (e.g. that are not excluded based on said correspondence to a cluster of the second plurality of subsets), in which said ranking combines a first score based on a size of the cluster and a second score based on the shape measure of the cluster. The ranking may thus be used to determine the selection, e.g. selecting the highest or the top-N (e.g. 2, 3, ...) highest ranked clusters. The ranking may, optionally, also include a further score based on the correspondence of the cluster with (e.g. the best match of) clusters of the second plurality of subsets.

In a device in accordance with embodiments of the present invention, the subset generator may be adapted to divide the first region, respectively the second region, into said first, respectively second, plurality of pixel subsets by forming a partition of the first region, respectively the second region, into blocks or patches based on a spatial distance metric and/or into subsets based on an image codomain distance and/or based on a combination thereof, such that each subset groups pixels together that are close in space and/or in value.

In a device in accordance with embodiments of the present invention, the subset generator may be adapted to perform an image segmentation (and/or other suitable processing) of a reference image and/or reference spatial information received via the input to determine said first and second regions, in which the reference image and/or reference spatial information is received via the input (11) as acquired by the camera, a further camera and/or a further spatial information source. For example, additionally or alternatively, the subset generator may be adapted to perform (e.g. as said other suitable processing or element thereof) an image subtraction between a reference image acquired with the subject present and a blank image acquired without the subject present, and/or applying a similar image background compensation technique, for use in determining the first region and second region, e.g. (optionally) in combination with an image segmentation of the image after the irrelevant background was removed.

A device in accordance with embodiments of the present invention may comprise an optimizer, in which the subset generator is adapted to repeatedly divide the first region into said first plurality of pixel subsets for different values of at least one optimization parameter representative of (at least) the size of the pixel subsets, and in which the optimizer is adapted to calculate and evaluate a quality metric based on the pixel subsets obtained in each iteration for a different value of the at least one optimization parameter so as to select the parameter value for which a sufficient or optimal value of the quality metric Is obtained. Thus, the selected parameter value(s) and the corresponding division of the first and second region into respectively the first and second plurality of subsets may then be used by the clustering processor and selector.

In a device in accordance with embodiments of the present invention, the optimizer may also use, as component of said at least one optimization parameter, a size of a temporal observation window used by said signal dynamics analyzer.

In a device in accordance with embodiments of the present invention, the quality metric calculated by said optimizer may comprise or consist of a signal-to-noise ratio.

In a device in accordance with embodiments of the present invention, the signal dynamics analyzer may be adapted to perform a Fourier transform in the temporal domain to obtain one or more temporal frequency characteristics for use in determining said at least one dynamic characteristic value.

A device in accordance with embodiments of the present invention may comprise a subset eliminator for pruning the first plurality of subsets based on predetermined criteria for one or more values determined on a subset-per-subset basis, so as to reject subsets that have a low likelihood of corresponding to homogeneous areas of the tissue of interest, and for pruning the second plurality of subsets based on the same and/or further predetermined criteria, and in which the clustering processor may be adapted to cluster respectively the pruned first plurality of subsets and the pruned second plurality of subsets.

In a device in accordance with embodiments of the present invention, the subset eliminator may be adapted to prune the first and/or second plurality of subsets based on the predetermined criteria comprising one or more criteria for: a mean pixel value per subset, a spread of the pixel values per subset and/or at least one value indicative of motion associated with the subset, such that the subset eliminator is adapted to reject subjects of pixels that show excessive motion, an inhomogeneous pixel value distribution and/or a pixel value that is, on average, outside a predetermined target range.

In a device in accordance with embodiments of the present invention, the subset eliminator may furthermore be adapted to apply a further pruning of the first and/or second plurality of subsets based on the at least one dynamic characteristic value, and in which the clustering processor is adapted to cluster respectively the further pruned first plurality of subsets and the further pruned second plurality of subsets.

In a device in accordance with embodiments of the present invention, the subset eliminator may be adapted to apply this further pruning, in which the further pruning is based on at least one predetermined criterion for the spectral energy of the largest spectral peak of the Fourier spectrum determined for each subset and/or based on a value determined therefrom.

In a third aspect, the present invention relates to a diagnostic imaging system with an examination zone, in which the system comprises a camera for acquiring images from a subject when undergoing an examination while positioned in the examination zone, and a device in accordance with embodiments of the present invention, which is operably connected to the camera to receive camera images from the camera as input.

In a fourth aspect, the present invention relates to a computer-program product for performing a method in accordance with embodiments of the present invention when executed by a computing device.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

FIG. 1 shows a method in accordance with embodiments of the present invention.
FIG. 2 shows a device in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a method for determining a photoplethysmography (PPG) signal indicative of a subject's physiology based on remote camera observation, and/or for determining an image region-of-interest (ROI) to extract such PPG signal from, e.g. by a prior-art method that receives the ROI (e.g. as an image map, as a mask image or as correspondingly masked images in a video stream) for in subsequently determining the PPG. Since methods to extract the PPG signal, once a suitable definition of the usable image content (e.g. a mask or other suitable description of the region of interest to use) is provided, are well-known in the field, such methods are not discussed in detail, but it will be understood that a method in accordance with embodiments of the present invention may comprise such well-known signal extraction step so as to obtain and provide a high-quality PPG signal (e.g. having a good signal-to-noise ratio, and/or robust against movement and/or other artefacts) based on the region-of-interest that is determined in accordance with embodiments of the present invention.

As known in the field, photoplethysmography (PPG) is an advantageously low-cost optical approach for detecting microvascular blood volume changes associated with the heartbeat dynamics. It can advantageously provide non-invasive measurements, e.g. using camera imaging. The raw PPG signal comprises a pulsatile component (i.e. an "AC" component) that is synchronous with the cardiac cycle, i.e. the PPG signal of interest to extract. Frequencies that are not indicative of the signal of interest can be easily removed by filtering, e.g. to remove a slow ("DC" and/or lower frequencies) component that is not relevant, e.g. components due to the average illumination conditions and/or potentially changes due to slow movement, respiration, thermoregulation (e.g. specifically when using imaging in the infrared domain) and/or other such factors. Higher frequencies outside the physiological range of interest, e.g. which may be substantially associated with noise, can equally be filtered out. Conventional signal processing techniques as known in the art (e.g. filtering and/or more advanced techniques, e.g. based on machine learning), can thus be applied to extract the useful PPG signal from the live video stream provided by the camera monitoring, once the set of pixels in the (and each) video frame from which the signal is to be extracted has been established, i.e. the set of pixels corresponding to the region-of-interest definition provided by a method in accordance with embodiments.

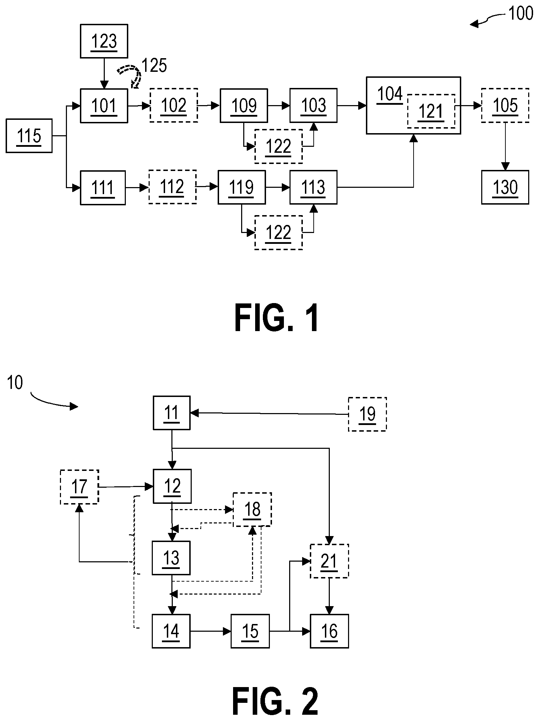

FIG. 1 shows an illustrative method 100 in accordance with embodiments. The method comprises acquiring 115 camera images, e.g. a stream of images, e.g. capturing a video or live stream, from a camera configured to monitor at least one body part of the subject. The camera images may be obtained by directly observing the subject, e.g. the subject may be in the direct line of sight of the camera(s), or indirectly, e.g. using one or more reflective surfaces in the optical path. Lenses and/or other optical elements may optionally be used to optimize the field of view of the camera, e.g. to magnify the relevant body part(s), to focus the image and/or the like.

The camera may be an infrared camera (e.g. operating in the near infrared range, NIR, e.g. a camera sensitive in the range of 800 to 850 nm), e.g. such that a PPG signal can be extracted without requiring an adjustment of the (visible) lighting conditions, and/or without being influenced by ambient lights (in the visible spectrum). Since the human eye is not sensitive to infrared light, the subject being monitored is therefore not affected by unpleasant, disturbing and/or uncomfortably (or even painful) bright light. However, embodiments are not limited thereto. For example, it is also possible to extract a PPG signal from imaging in the visible light range. For example, the light absorption of (de)oxygenated hemoglobin is stronger in the (near) infrared wavelength range than for (e.g. red) visible light, and infrared light can penetrate deeper into skin (e.g. the influence of melanin in the skin is less pronounced). Also, infrared cameras, e.g. based on semiconductor pixel detectors, can have a higher sensitivity in the NIR range (e.g. 800-850nm) than for wavelengths above 900 nm, which can result in a higher signal to noise ratio and/or better operation when the body part of interest is not ideally illuminated. Other light ranges (than IR or NIR) may also be used for PPG signal detection, since it has been observed that video imaging in, for example, the red or green wavelength range can also provide sufficient information to detect the slight variations in intensity that are used.

The method may comprise acquiring camera images from a camera configured to monitor a body part of a subject during a medical and/or diagnostic examination or intervention. The examination may be, for example, a magnetic resonance imaging, computed tomography, positron emission tomography or single-photon emission computed tomography examination. Examples of interventions include therapeutic and related procedures, such as surgery and radiotherapy. Embodiments may also find application in general-purpose subject or patient monitoring, such as in an intensive care unit, a patient ward, or in a non-medical setting, e.g. in a nursing home. Particularly, the PPG signal determined by the method may be used to monitor the health and/or state of the subject, and/or to control a system or device used for an examination or invention. The body part(s) of interest may comprise the face or a part thereof, e.g. the forehead, but is not necessarily limited thereto (e.g. also the skin of another body part may be used, e.g. of the arm, the chest, a leg, the abdomen, ...).

The method comprises dividing 101 at least one first region in the camera images, e.g. in a reference image, into subsets of pixels, e.g. patches. The subsets of pixels, e.g. patches, may form a partition of the first region (e.g. such that the entire first region is covered by the union of the nonoverlapping patches). However, the latter is not necessarily essential, e.g. some overlap between patches is not necessarily excluded.

Furthermore, in addition to the step of dividing 101 the first region into patches, a second region in the camera images is divided into subsets of pixels (e.g. patches) as well, in accordance with embodiments of the present invention. The first and second region are different image regions, e.g. typically disjunct (and non-empty) parts of the image, e.g. such that no overlap exists between the first and second region, each region is not equal to the entire image, and each region is not empty. However, some insubstantial overlap may exist, e.g. in so far that this is negligible, such as pixels of a shared border contour between both regions or the like. For example, the intersection between both regions may be less than 10%, preferably less than 5%, even more preferred less than 1%, e.g. substantially 0%, relative to the union of both regions in size, e.g. when evaluating a ratio of the number of pixels in the intersection over the number of pixels in the union.

The first region is representative of image content where the tissue of interest is presumed to be present, whereas the second region is representative of image content where the tissue of interest is presumed to be absent, e.g. is less likely or unlikely to be found.

The union of the first and second region may form the entire image, e.g. the two regions may partition the image or may cover the image entirely. However, this is not necessarily the case. For example, background image content (e.g. regions of the image where the subject is unlikely to be present) may be ignored, whereas the first region may define a zone of the image where the tissue of interest (e.g. exposed skin) is likely visible (in a suitable form, e.g. sufficiently lighted etc.) and the second region may define a zone where the tissue is likely not clearly visible (but where image content is not necessarily independent of the subject as such). Such zone where the tissue of interest is unlikely to be clearly visible may include parts of the subject's body that are covered by clothing, glasses, a blanket or such, where a clear view is potentially obstructed by equipment, and/or where body features are more likely to be found that could be a source of errors in inferring the PPG signal, e.g. the eyes, the nose, hair, fingers, nails, a belly button, nipples, skin folds and/or other such features.

A more conventional "living skin" approach, in which patches of the image are analyzed for useful signal content, can thus be extended in accordance with embodiments of the present invention by analyzing a first region with likely relevant image content as well as a second region with likely irrelevant image content. Therefore, in addition to analyzing candidate living-skin patches, patches of the (a priori known, i.e. presumed) non-skin areas are also taken into account.

For this initial area division step, a reference image may be used, which may be a first image upon starting the procedure, a random image from a (e.g. short) sequence of images, an image selected therefrom based on a quality metric (e.g. to exclude effects due to an initial stabilization of the camera acquisition process, e.g. autofocusing, gain calibration and/or the like), and/or may be based on an aggregation of several images, e.g. constructed from a plurality of (a short sub-sequence of) the camera images, e.g. by averaging or the like. However, since the subject and camera may be considered to be substantially static (i.e. the camera is preferably fixed and motion of the subject is preferably avoided), the selection of such reference image may not necessarily be essential, e.g. the division into patches may be considered to be, at least roughly, applicable to any subsequently (and/or previously) acquired image frame in the same session as well, and, if entirely based on predetermined assumptions (e.g. when always using the camera in the same spatial configuration and always positioning the subject in the same manner with respect to the camera for a session), even for any session as such.

The first region, and/or a fixed set of patches covering the first region, may be predetermined, e.g. may be defined as function of a location where the tissue of interest (of the subject's body) is presumed to be, approximately, present. The second region, and/or a fixed set of patches covering the second region, may be predetermined as well, e.g. defined as function of a location in the image(s) where the tissue of interest is presumed to be absent. Likewise, the patches may be defined in a fixed manner, e.g. a predetermined partitioning of a predetermined first region (resp. the second region) of camera images, regardless of the session and/or subject to which the procedure relates. In other words, a predetermined or case-per-case (independently) determined mapping of the camera pixel coordinates (e.g. the image grid) may be used to determine the first region and second region, and/or the patches of each region, without requiring a reference image as such. The region definition and/or patches definition (per region) may be established independently from specific camera image(s), and may thus be considered to apply to any acquired image, e.g. to each single image frame in a sequence obtained to extract PPG information from, by definition. The region definitions and/or patches definitions may be entirely based on prior knowledge of the camera (and subject) setup, and/or may be determined independently (e.g. based on another camera system and/or spatial data acquisition system).

This may be particularly advantageous in an application where the camera is generally fixed, or is positioned, oriented, focused, zoomed and/or otherwise configured, using predetermined reference frames and/or parameters (e.g. a fixed arrangement in a scanner room and/or a system that uses a motorized/automated configuration), and the subject is generally positioned, oriented, etc., in the same or generally a similar way with respect to the camera, or with respect to a reference frame with a known relation to the camera's reference frame (possibly taking parameters as discussed hereinabove, such as automatically controlled camera orientation, position and/or the like, into account).

For example, in an illustrative application of usage in a medical imaging room (or other medical facility where a patient is to be monitored), the camera may view a known area (or, more accurately, a cone in space) of the room and the patient may be placed in a known position in the room (possibly taking parameters of the medical imaging system, such as patient table translation and/or the patient's pose for the procedure into account), such that an approximative region in the camera's images in which the tissue of interest is located can be easily inferred, and, likewise, the second region where the tissue is likely not present (or is presumably not imaged sufficiently clearly) can be determined as well.

The first region (and/or the set of patches dividing the first region) and/or the second region (and/or the set of patches dividing the second region) may alternatively be determined by an image segmentation or similar image processing technique. For example, a simple, e.g. two-component, segmentation may be performed to separate the body and/or the body part(s) and/or the tissue of interest (e.g. exposed skin) of the subject (first region) from its background (second region). A three, or more, component segmentation may be used to detect the skin (or other tissue of interest, i.e. the first region), (an) irrelevant part(s) of the body (second region) and (a) background image region(s) (e.g. the room and/or equipment; i.e. forming a third component and/or a number of further components that may be entirely ignored). Other segmentation approaches are not necessarily excluded, e.g. where the expected image content is used to predefine a number of components that can be visually (and algorithmically) discerned, in which at least one of which (not excluding combinations) can be associated with the first region and at least one (or more) of which can be associated with the second region. For example, a plurality of image segments (that is, more than two) may be expected based on assumed pixel properties (e.g. intensity values etc.), and explicitly taking such inhomogeneous second region and/or (an optional, further ignored) background region into account (by accommodating additional segmentation image segments) may advantageously improve the quality of the image segmentation that is obtainable.

As another example, a blank image (without the subject present) of the same environment may be used to determine the first region by background subtraction and define the second region as its complement (possibly including normalization procedures and/or more advanced approaches). A combination of background subtraction and segmentation, and/or other image processing techniques, may also be used. For example, a "blank" background image may be subtracted to remove static image components, and a segmentation may be performed on the result thereof to identify the first region, comprised of the tissue of interest (e.g. exposed skin), and the second region, comprised of other nonstatic and/or variable elements in the image, such as covered parts of the body, hair, the eyes, equipment (e.g. tubes, cables, a breathing mask, etc.) and/or other such irrelevant image content, particularly where some dynamic changes in the image content might be expected in this second region, as opposed to the static content removed by the image subtraction. It will be understood that the image subtraction referred to hereinabove may be implemented by any equivalent approach that is more complex than merely a simple arithmetic matrix subtraction, e.g. taking normalization of the images onto account and/or applying, in general, any suitable background compensation technique.

The image segmentation (or other suitable image processing) is not necessarily limited to images obtained by the camera system, or necessarily uses (only) the images used for the subsequent PPG extraction to be performed. For example, a further camera may be configured to provide a better image contrast and/or other advantageous properties that allow the subject (more generally, the body part or tissue region of interest) to be numerically separated from non-relevant parts of the subject, e.g. clothed or covered parts of the body. For example, the camera(s) used for the PPG signal extraction may be optimized to provide a good sensitivity for detecting the subtle PPG signal of interest (e.g. in the infrared spectrum and/or another specific part of the spectrum, e.g. a green light band), but is not necessarily ideal for segmenting the image, e.g. to discerning the tissue of interest, such as skin, from other image content. Thus, another camera (or cameras), more suitable for this purpose, may be used, e.g. a color camera or a depth camera, in so far that the segmentation result can be transformed to the reference frame of the camera used for the PPG signal extraction. It will be understood that even when a deterministic relationship between the camera reference frames is not a-priori known, other techniques may be used to transform the segmentation map, e.g. using minimization of mutual information between an image obtained by the PPG camera system and an image obtained by the further camera system used for image segmentation (and/or for determining the first and second regions by similar processing techniques). Likewise, the source of information for inferring the first and second regions, e.g. by segmentation, is not necessarily limited to a camera system, in the narrowest sense, but may comprise or consist of any suitable means for acquiring spatial information, including, for example, a medical imaging system, e.g. which may be intended to be used during the PPG monitoring of the subject.

Whereas reference is made to segmentation, it will also be understood that this does not necessarily exclude more advanced approaches, such as fitting a geometrical model of the subject (or the relevant body parts or parts of the subject) to the image data and/or evaluating a trained machine learning model to determine a suitable (approximative) segmentation (i.e., in general, a descriptor of the first and second region, and/or, directly, of the patches forming each of said regions) as output.

In a "living skin" procedure, and/or a similar procedure, it is typically assumed that the pulsatile component of interest, i.e. the PPG signal, stands out from the measurement noise, or, in other words, that it is sufficiently detectable and discernable in the acquired camera pixel signals (e.g. after suitable processing). Particularly, an averaging operation (or, in general, applying a suitable summarizing calculation, e.g. a measure of statistical centrality) over the different pixels of each patch should sufficiently reduce the sensor quantization noise and/or other noise components in order to be able to discern the usable signal content (and the patches associated therewith) from the noisy content in irrelevant and/or unsuitable patches where the PPG signal is too weak, polluted by heavy noise, confounded by other, irrelevant signals or simply not present. Furthermore, as discussed in detail hereinbelow, techniques to analyze the dynamics of the signal (changes over time) are typically applied, such that a sufficiently long observation time is required to be able to detect the signal component of interest. For example, a suitable window size of a Fourier analysis may need to be selected, but without losing too much temporal resolution and without increasing the sensitivity to errors too much.

Instead of using a predetermined (and potentially arbitrary) patch size in the step(s) of dividing the first area (as well as the second area) into patches, the patch size and/or time observation window used for analysis of signal dynamics (e.g. Fourier window) may be optimized in accordance with embodiments of the present invention, e.g. so as to obtain a good the signal-to-noise ratio (SNR). For example, temporal fluctuations may be present in the raw pixel signals that may be attributable to the illumination conditions (e.g. when natural lighting or a low-quality light source is used), to stability of the environment, and/or to motion. Also, physiological differences (intra-subject and/or between subjects), such as natural heart rate variability may influence an optimal choice of the patch size (and/or time window) from one session to another. Therefore, inherent (cardiac cycle related) and exogenous (e.g. lighting conditions) differences between sessions, e.g. between different applications of the method (such as for use in detecting PPG signals for different patients), at different times and/or in different environments, may lead to the (e.g. optimal or optimized) parameter selection to be session-dependent. An optimization of such parameter(s) in accordance with embodiments may thus advantageously improve accuracy, robustness, stability and/or, generally, the processed PPG signal output quality.

The optimal, or at least a good or suitable, selection of a temporal observation window, e.g. a FFT window selection, as well as the choice of patch size may, for example, depend strongly on the heartrate and heartrate variability to be expected for each individual case, which, unfortunately, may be unknown (or not accurately known) a priori. In fact, a goal of the method in accordance with embodiments may very well be to accurately measure and/or monitor this heart rate and/or other cardiac parameters. While a larger patch size may be preferable in some circumstances, e.g. when a shorter FFT window is used and/or to reduce the noise by (spatial) averaging, a patch size that is too large could also be disadvantageous. The probability of finding homogeneous patches (e.g. that substantially exclusively correspond to exposed skin area) may decrease as the patch size increases. Therefore, it will be understood that an optimum may exist between these extremes, which is not a priori known and dependent on each specific case.

Where reference is made to the patch size, it will be understood that the patch size is not necessarily strictly constant over all patches for a specific choice of the patch size parameter. In other words, some variation in the patch size may be possible for a specific patch size parameter. For example, the step of dividing the first region (and/or the second region) into patches may be implemented in a way that allows some variation, e.g. such that the size parameter more accurately determines the average (or median, ...) patch size to be used (in the iteration for that parameter choice).

Even if a fixed (and relatively simple) approach for dividing a region into patches with a predetermined and deterministic (uniform) patch size is used, e.g. partitioning the region by a Cartesian block pattern of the given block size, an unregular boundary of the first/second region may still cause some edge regions to contain less (or more) pixels than a typical, more central, (block-shaped) patch.

However, if so desired, for example when a processing technique is used that relies on (or is sensitive to) a uniform patch size (number of pixels per patch), a uniform patch size (per iteration, for a corresponding specifc choice of patch size) may be (optionally) enforced. For example, the (first/second) region definition may be tuned so as to fit all the patch sizes to be considered without splitting any patch near the boundary, e.g. by defining the regions at a resolution (scale) corresponding to the smallest common multiple of the different patch scales to evaluate. In another example, since the first (respectively second) region does not necessarily correspond exactly to the tissue of interest (respectively, to the irrelevant image content), e.g. the region may be merely a best guess based on the available a-priori information, each (uniformly sized) patch, e.g. block, may also simply be assigned in its entirety to either the first region or the second region, e.g. based on majority voting, without much issue and/or substantial consequence.

Thus, since the PPG signal of interest and the noise components (and/or, generally, nuisance contributions) may vary across sessions, e.g. at least due to differences in each subject's cardiac response pulsatility, in a method in accordance with embodiments of the present invention, the size of the patches (e.g. used for the steps of dividing 101,111 the regions) and/or a time window size (e.g. FFT window size) for analyzing temporal changes may be selected 123, e.g. optimized and/or tuned, based on the acquired image data (directly or indirectly), e.g. so as to be above the noise floor but sufficiently small to avoid or reduce disadvantageous effects associated with averaging over larger areas and/or analyzing the signals over longer time frames. Such parameter selection 123 may be implemented in various ways.

For example, at least the step of dividing 101 the first region into patches (and optionally also dividing 111 the second region into patches and/or a further step or steps as discussed hereinbelow), may be repeated 125 for different values of at least one optimization parameter representative of the size of the pixel subsets, e.g. repeating the step or steps for different patch sizes. Other parameters may be tuned as well (e.g. additionally to the size optimization), such as a time window size for the signal dynamics analysis (e.g. Fourier analysis). After each iteration, a quality metric is calculated, (directly or indirectly) based on the acquired image data as organized in the set of patches for that parameter choice, e.g. based on an intermediate output of the step or steps that are performed in the (e.g. in each) iteration and/or based on the region-of-interest output thus obtained in the iteration, so as to indicate the fitness of the tested parameter selection.

For example, substantially the entire procedure, e.g. to the point that the region-of-interest is determined, may be executed in each iteration, and the quality metric may be determined directly from the obtained region-of-interest for a specific parameter selection (and/or from the PPG signal extracted from a video stream acquired by the camera using that ROI mask). Thus, the best region-of-interest, as indicated by the quality metric, may be selected from a plurality of trials with different parameter selections, and/or the parameters may be tuned for use in each subsequent iteration based on the output of the previous iteration or iterations. Without limitation thereto, the quality metric may comprise an estimate of the signal over noise ratio (SNR) of the extracted PPG signal for the obtained ROI, and/or may be more complex, e.g. taking various factors into account, such as the ROI size, SNR, the signal contrast and/or one or more known measures of (generic) signal quality, and/or of PPG signal quality (specifically).

In an advantageously efficient approach, a simpler quality metric may be used that does not require the execution of all steps, e.g. a quality metric that can be easily calculated based on intermediate results, e.g. based on the patches obtained by only the region division step(s), e.g. based on the patches after applying the pruning steps discussed hereinbelow, or based on the dynamic parameter estimations (e.g. Fourier analysis) steps discussed hereinbelow. It will be understood that the area partitioning (region division 101 and/or 111) step(s) and/or the pruning step(s) may be performed relatively efficient, compared to the more computationally intensive signal dynamics analysis (Fourier analysis) and/or steps dependent thereon. Thus, advantageously, the quality metric may be efficiently calculated based on the pruned set(s) of patches (first region or first and second region), without taking the dynamics into account. However, e.g. particularly when the temporal analysis window is also to be optimized, the quality metric may also be based on intermediate outputs of further steps, e.g. taking the characteristic(s) of signal dynamics determined for the (e.g. pruned) patches into account. For example, the signal dynamics may be characterized relatively efficiently, e.g. using dedicated Fourier transform hardware, multiprocessor and/or GPU, such that the use of such information in the (e.g. iterative) parameter optimization would not be necessarily prohibitive.

Furthermore, in the optimization step, instead of a high-quality temporal dynamics analysis as might be used in the further steps of the method discussed hereinbelow, e.g. instead of calculating the Fourier spectrum entirely (in detail), a simplified or approximative analysis of temporal dynamics may be used as substitute, e.g. by detecting a signal component or components for only a specific frequency or a few specific frequencies and/or for only a specific spectral band or a few spectral bands. For example, such limited information may be sufficient to calculate a quality metric (or to be used as an element in the calculation thereof), e.g. to give at least a rough indication of the signal quality with respect to the expected temporal dynamics. To illustrate this, the spectral power for a predetermined frequency (or frequency band) may be quickly and/or easily determined, in which this frequency (band) is presumed to contain at least some cardiac-cycle-related activity (e.g. based on an average heartrate), whereas in the more detailed analysis discussed hereinbelow (e.g. after the parameter selection), the peak frequency and peak signal strength may be determined in detail by more demanding calculations.

Variations on this approach can easily be envisioned, such as normalizing the spectral power (or other suitable value) for a presumed relevant frequency (band) by a similarly calculated value for a frequency or band where no relevant (i.e. cardio-related) activity is to be expected.

In another illustrative approach, a scale-space analysis may be used, e.g. in which the signal is down-sampled (in the time dimension) by various factors (e.g. a scale pyramid of powers of 2), such that the average, standard deviation and/or other such measures of the signal (e.g. already averaged per patch) can be used as a proxy for the signal dynamics in different (approximative) frequency bands (e.g. dependent on the corresponding time scale).

The quality metric used for the parameter selection (e.g. optimization) 123 may generally express the fitness of the parameter selection, e.g. the patch scale and/or the time analysis window, for which it is calculated. Thus, the parameter choice(s) corresponding to higher (or the highest) quality metric value(s) can be selected for further use in determining the region-of-interest, from which the PPG signal of interest can eventually be extracted. Obviously, the quality metric can be alternatively defined, entirely equivalently, as a cost function, e.g. such that lower values indicate the more suitable parameter choices.

The quality metric may, for example, have a maximum (or, equivalently, a corresponding cost function may have a minimum) when a sufficient (e.g. at least a certain predetermined) number of patches (generally, subsets of pixels, i.e. "real," spatially connected, patches and/or "virtual," aggregated, patches, as discussed hereinbelow) render a consistent signal strength within an expected (e.g. predetermined) range. The signal strength may be estimated as an approximative indicator of the PPG signal strength, e.g. based on the mean pixel value and/or deviation, on the largest spectral peak magnitude values, and/or on an intermediate result of the method discussed further hereinbelow, or may be directly estimated, e.g. extracting the (e.g. a short run of) PPG signal using the parameter selection at hand to estimate the signal to noise ratio of the PPG signal for that parameter choice directly.

The expected predetermined range of the signal strength may be determined by the expected PPG signal properties in view of physiology. This may be implemented, without limitation thereto, by testing the signal strength value for each patch to a normal distribution, e.g. calculating the Z-score of the mean value (e.g. the signal strength, e.g. the largest spectral peak magnitude) associated with each patch against a normal distribution with parameters (mean and standard deviation) corresponding to the expected distribution (the physiologically expected signal), and summing (or averaging, or in another suitable manner combining) the obtained Z-scores over all patches in the region (first region).

Thus, a total score is obtained that increases as more patches fit the expected curve better. Many alternatives can be considered, e.g. testing an ensemble of (pixel-based) values in the patch, instead of e.g. a mean value of the patch, against the known (i.e. presumed) distribution, for example by using a Student's t-test or F-test to compared the sample against a target distribution. Other types of distribution to test against may also be used (including, potentially, an empirical, e.g. histogram-based, distribution). Furthermore, various alternatives to the per-patch or per-pixel characteristic on which such scoring and/or testing is based can be considered, e.g. a signal strength, a pixel intensity, a signal-to-noise ratio, a spectral peak magnitude, a spectral peak magnitude ratio, etc.

The use of a pixel intensity value (or averaged per-patch intensity value) advantageously may allow a quality metric to be calculated after only requiring the step of dividing the first region (and optionally also the second region) to be executed for each specific parameter selection to be tested. This may thus be performed very efficiently (specifically in multiple parameter test iterations). In another illustrative approach, the pruning step may also be performed for each iteration to reject irrelevant patches, and/or the step of calculating (a) characteristic(s) of the signal dynamics may be performed as well, e.g. such that the signal dynamics per patch can be taken into account in the parameter selection process (e.g. to calculate the quality metric).

In a relatively simple approach, the region-of-interest may be determined for each parameter space point over a range of the parameter (e.g. the patch size) or over a parameter grid of the parameters (e.g. the time window and patch size), and the best region-of-interest may be used as output and/or for PPG signal extraction, i.e. a simple line/grid search optimization may be used. The parameter points may be equidistantly sampled along the line or over the grid (e.g. in a Cartesian grid scan), or may vary (e.g. sampling heavier in regions of the parameter space where an optimal choice is overall more likely to be found). A search strategy may optionally take the properties and/or specific nature of the parameter(s) into account, e.g. using a multi-resolution and/or hierarchical search to find the optimal (or at least sufficiently tuned) parameter(s). It will be understood that, when more than one parameter is being optimized, the combination may be jointly optimized (e.g. as a parameter vector to optimize) or separately (e.g. in a nested optimization procedure).

More advanced techniques may use the results from the previous iteration or iterations to determine a next sampling point, e.g. using an estimate of the parameter gradient(s) and/or the parameters' Jacobian matrix.

In yet another strategy, a first run, or a few first runs, may be used to estimate an initial region-of-interest, from which the heart rate may be estimated (e.g. using the ROI for extracting the PPG signal over a short time window), after which this information may be used to obtain a better estimate of a suitable parameter selection, from which the procedure restarts to obtain a better ROI definition. This can be repeated multiple times, if deemed necessary, or only for one refinement step. It will be understood that signal noise may also be taken into account, e.g. using the heart rate and an estimate of noise (and/or other quality measures derivable from the observed PPG output and/or ROI) to look up a suitable parameter selection in a lookup table, by an empirical relation or the like. Alternatively, a machine learning strategy may be used, e.g. to propose a parameter space optimum based on the output of a previous run (e.g. the ROI obtained for a previous parameter selection, i.e. in an iterative approach) and/or an input vector describing the specifics of a use case, e.g. patient's demographic data, room conditions, time of day, information on the procedure in which the PPG extraction is applied (e.g. an identifier of a medical imaging procedure), and/or other data that could be implicitly used for inferring the suitable parameter selection by an algorithm trained on appropriate examples.

While embodiments may typically divide the first/second region into abutting, continuous areas, e.g. forming a partition of singly-connected image patches, some variations and/or alternatives may be envisioned. For example, instead of, or in addition to, partitioning the region into singly-connected areas (patches in the more conventional sense), alternatively (or additionally) different pixels may also be grouped together when similar in pixel value instead of being spatially close together. Spatial grouping and value-domain grouping of pixels may also be combined to form "patches," in which each patch is comprised of pixels that are close together in space, without necessarily requiring a predetermined shape (e.g. a block, square, rectangle, ...) or connectedness, but also relatively close together in value space (i.e. of the same or similar pixel value).

For example, a (spatial) distance metric (not necessarily Cartesian, e.g. a Manhattan norm; without limitation to these examples) may be combined, e.g. by a weighted sum, with a (value space) distance metric (e.g. the absolute value of difference in pixel value, without limitation thereto), and the (first, respectively second) region may be divided into pixel sets to optimize the combined metric, e.g. so as to obtain a target number of pixels per set while minimizing the metric.

Use of a value-space (the image codomain) distance, exclusively or in combination with spatial (the image domain) distance, to determine the patches for each region (generally, to group pixels into a plurality of subsets) can be particularly advantageous when applied in a parameter optimization strategy as discussed hereinabove, but is not necessarily limited thereto. For increasing patch sizes, e.g. an increasing number of pixels per subset "patch" (at least on average), the likelihood of forming an inhomogeneous patch increases, e.g. it becomes less likely that a single pixel group (the subset or patch) contains only the tissue of interest (e.g. skin). Thus, grouping pixels into patches in solely the spatial domain is naturally limited for larger patch scales, since above a certain size the quality of patches deteriorates due to pollution of the desired, e.g. skin-related, signal by pixels assigned to the same patch that have poor or unrelated signal content.

For larger patch sizes (i.e. subset sizes), it may be preferable to switch from pixel combinations based on spatial neighboring to another metric based on the pixel output value, e.g. an intensity-based metric. Such switching can be a discrete switching, but may also be continuously tuned by varying weights of a combination metric of spatial and value distance.

Alternatively, patches may be formed based on a spatial neighborhood, but, e.g. beyond a predetermined size threshold, larger patches may be formed by combining smaller (spatial-neighborhood-based) patches based on the alternative value-space metric, e.g. selecting four blocks of primitive patches based on similarity in pixel value, without necessarily requiring spatial proximity. The latter may allow an efficient implementation, e.g. the native (smaller) patches may be formed by dividing the image region at hand into blocks (e.g. forming a Cartesian grid over the region) at very low computational cost, while these primitive patches may be combined into subsets of the desired (larger) target size based on their intensity, which can also be performed at a reasonable computational cost (e.g. in view of simple calculations performed on a relatively small number of entities, i.e. the primitive patches). For example, without limitation thereto, suitable combinations of patches may be found by only using calculations on a relatively small matrix of NxN (or even only the upper/lower triangle part thereof in view of the matrix symmetry) of absolute average (per primitive patch averaged) pixel intensity differences between pairs of primitive patches, in which N represents the relatively low number of primitive patches.

Where reference is made to an illustrative intensity-based metric, it is noted that this may refer, in general, to any suitable value-space (e.g. codomain) metric. For example, the camera may be configured to gather vector data (e.g. a color image), and the value-space similarity metric may be evaluated based on vector pixel values, and is thus not necessarily limited to a scalar comparison of intensity values. Also, the similarity measure is not necessarily limited to only considering a measure of centrality (e.g. average intensity), but may use a comparative measure of (an)other property(-ies) of the set of pixels (alternatively or additionally) as well, e.g. a standard deviation, a signal-over-noise ratio (in the spatial sense, e.g. mean intensity over standard deviation), higher-order statistical moments, texture metrics, and/or any other spatially descriptive measure. For example, for each pixel (or "primitive patch"), a vector may be formed that comprises different components indicative of the pixel value and/or the pixel values in a local (spatial) neighborhood of pixel (e.g. a primitive patch as discussed hereinabove), and pixels (or primitive patches) may be clustered based on similarity of this descriptive vector.

When applying a parameter tuning strategy as discussed hereinabove, it may be particularly advantageous to switch, discretely or continuously, from forming spatial domain neighborhoods (patches in the narrowest sense) to forming value domain neighborhoods (patches in the generalized sense of subsets). It will also be understood that a continuously varying combination of both types of metric, e.g. by a weighted sum, may be more appropriate for some optimization algorithms and/or may avoid missing a part of the parameter space where a parameter optimum would be found if the discrete switching point between metrics was postponed to a higher size threshold and/or the metric switch was executed in the, in this case, more preferable continuous form. However, it is also noted that an approach such as discussed hereinabove, in which primitive (solely spatial-neighborhood-based) patches are joined, when the target size requires so (e.g. beyond a certain target size threshold), into larger patches based on intensity (generally, on distance in the image codomain or a related space) may be easier to implement and/or advantageously more efficient. It is also noted that the use of a clustering technique, e.g. as known in the field of image processing, to group pixels (or primitive patches) together based on their value-domain properties may suffice to ensure, to some reasonable extent, a spatial cohesion of the patches (generally, subsets) that are obtained, even when the spatial neighborhood metric is no longer explicitly taken into account (e.g. is implicitly implemented and accounted for by the clustering algorithm).

To illustrate the approaches discussed hereinabove, consider a scenario in which a plurality of patch sizes is evaluated, e.g. patches of 10x10, 15x15, and 20x20 (or their equivalent approximative number of pixels). Suppose that a further increase of patch sizes would imply the unfavorable situation that (almost) all patches were to include an appreciable amount of non-skin area (for the partitioning of the first region). In a naive approach, a further increase in (spatial-neighborhood-based) patch size would therefore be prohibited, e.g. would not render the desirable result of finding a useful ROI (or a higher-quality ROI than found for the 20x20 test case). Instead of increasing the patch area as such, e.g. to 40x40, smaller patches may be combined into larger virtual patches, e.g., combining four 20x20 patches into one virtual (potentially spatially segregated) patch of, in total, 40x40 pixels. Such combination of patches can be obtained by a clustering technique, e.g., by extracting features of average level and standard deviation over the patch (possibly over the total observation period, e.g. averaging/computing over a predetermined window of time as well). Then, the selection of a suitable scale (patch size) can be done by selecting the size for which a sufficient number of (real or virtual) patches render a consistent signal strength within the expected range (as defined by physiology) and/or another suitable quality metric.

After the step of dividing 101 the first region into patches, and optionally after optimizing the patch size and/or other parameters, the first region's patches may be pruned 102 based on predetermined criteria for one or more values determined on a patch-per-patch basis. This may include the selection of patches that have a mean pixel value, spread and/or motion value (e.g. magnitude, motion vector components, and/or one or more metrics representative of motion) in a predetermined (corresponding) range(s) and/or satisfy predetermined criteria. Motion values, such as a magnitude of motion, vector components of a displacement vector, and/or other such metrics representative of motion may, for example, be determined by applying an optical flow algorithm (e.g. using two or more images obtained by the camera at different points in time, e.g. a short dynamic sequence).

The set of patches may thus be pruned to select a subset of patches thereof that are substantially static in position (little or no detectable movement), substantially well-lit, and/or substantially homogeneous (e.g. have a low standard deviation of the pixel values that form the patch). Where reference is made to average, spread, standard deviation and/or the like, it will be understood that the same effect can be obtained by various similar statistical measures of centrality and/or dispersion, e.g. median values, inter-quartile values, variance values, etc. Likewise, other simple, per-patch statistics (or other representative values) may be used, e.g. higher-order statistical moments, skewness, kurtosis, texture and/or pattern-based values, spatial gradients, and/or other such values, to prune patches that show properties that are not congruous with the desired tissue of interest and/or reflect undesirable imaging conditions, i.e. on a local (per-patch) basis.

Furthermore, after the step of dividing 101 the second region into patches, the second region's patches may also be pruned 112 based on predetermined criteria for one or more values determined on a patch-per-patch basis. These criteria may be the same as for pruning the first region's patches, or may differ. This means that some variations can be considered in implementation. For example, the values of interest may alternatively be calculated over the entire image (but on a patch-per-patch basis nonetheless), or over a union of the first and second region (but patch-per-patch). By applying some bookkeeping, flagging or other suitable approach, which patch corresponds to which region can be easily tracked. Depending on the specifics of the embodiment (e.g. hardware that is used, the choice of values to use for pruning and/or other such factors), it may be more efficient to calculate the values of interest over all patches or separately per region (possibly in parallel). It is also noted that even if not the same criteria are applied to patches of respectively the first and the second region, it may, under some circumstances, still be more efficient to calculate the values to which the (combined set of) criteria relate over all patches, e.g. by advantageously using parallelization technology (e.g. a graphics processing unit, GPU, and/or general purpose graphics processing unit, GPGPU), such that only different comparisons, thresholding operations and/or other criterion evaluators need to be applied to the patches of the two different regions.

Pruning 102, 112 the patches of the first and/or second region may include the selection of patches that have a mean pixel value, spread and/or motion value (e.g. magnitude, motion vector components, and/or one or more metrics representative of motion) in a predetermined (corresponding) range(s) and/or satisfy predetermined criteria. Motion values, such as a magnitude of motion, vector components of a displacement vector, and/or other such metrics representative of motion may, for example, be determined by applying an optical flow algorithm (e.g. using two or more images obtained by the camera at different points in time, e.g. a short dynamic sequence).

The sets of patches may thus be pruned to select those patches (per region) that are substantially static in position (little or no detectable movement), substantially well-lit, and/or substantially homogeneous (e.g. have a low standard deviation of the pixel values that form the patch). Where reference is made to average, spread, standard deviation and/or the like, it will be understood that the same effect can be obtained by various similar statistical measures of centrality and/or dispersion, e.g. median values, inter-quartile values, variance values, etc. Likewise, other simple, per-patch statistics (or other representative values) may be used, e.g. higher-order statistical moments, skewness, kurtosis, texture and/or pattern-based values, spatial gradients, and/or other such values, to prune patches that show properties that are not congruous with the desired tissue of interest and/or reflect undesirable imaging conditions, i.e. on a local (per-patch) basis.

For example, patches for which (substantial) motion is detected may be rejected (pruned away) in both the first region and the second region, e.g. to select (sufficiently) static (i.e. non-moving) patches. Patches in which (substantial) variation (over intra-patch pixel locations) is detected may be rejected (pruned away) in both the first region and the second region, e.g. to retain homogeneous patches. Patches that are over- or under- illuminated may be rejected for the first region, and optionally also for the second region, e.g. such as to select patches that fall within a suitable range of the dynamic range of the camera system. These pruning criteria may be applied by a logical OR operation, e.g. satisfying one rejection criteria may suffice for a patch to be rejected, or, entirely equivalently, retention criteria may be applied by a logical AND operation, e.g. only selecting patches to be retained for further processing when all of the selection criteria (e.g. logical negations of the aforementioned rejection criteria) are satisfied. Other choices of criteria and/or values to evaluate such criteria against, and/or of strategies to combine the result of multiple criteria (e.g. by logical operations, by majority voting, ...) are also possible in accordance with embodiments, e.g. as the skilled person is able to decide on by relying on the knowledge in the field and straightforward implementation, testing and/or simulation.

The method furthermore comprises determining one or more dynamic characteristic values, e.g. characteristics, that are indicative of temporal dynamics, on a patch-per-patch basis. Thus, this/these value(s) are based on multiple camera images corresponding to different points in time, e.g. a sequence (time series) of camera images. These dynamic characteristic values are determined 109 for patches of the first region, as well as determined 119 for patches of the second region. Particularly, the characteristic(s) that is/are determined 109, 119 per patch may be the same (e.g. of the same type, implemented in the same way, and/or corresponding to the same physical quantity), e.g. the same dynamic characteristic values may be determined 119 for patches of the second region as for patches of the first region. This allows the results obtained for this patch-per-patch dynamic characteristic in the second region to be taken into account when processing the same characteristic obtained in the first region, as will be explained hereinbelow.

For the sake of efficiency, these dynamic characteristic values may be determined 109, 119 after pruning 102, 112 the patches of respectively the first and the second region, e.g. so as to avoid calculation of the dynamic characteristic value(s) for patches that were rejected (pruned away). However, this is not necessarily the case. For example, parallelization technology may be used to quickly and efficiently calculate the dynamic characteristic(s) for all patches (but still on a per-patch basis), even if the values that are obtained for patches that are/were/will be pruned away are essentially ignored.
The method comprises clustering 103 the patches (e.g. patches which were not withheld by the pruning step) of the first region based the one or more dynamic characteristic values (representative of a dynamic characteristic of the patch), and, likewise, clustering 113 the patches of the second region based on the dynamic characteristic value(s). This clustering 103, 113 may optionally be performed for (only) the patches that are not pruned away (i.e. exclusively taking the patches into account that were not rejected by the pruning). The clustering may be performed separately (e.g. independently) for the patches of the first region and the second region.

Again, it is noted that in accordance with some embodiments, it may be acceptable to apply the clustering to all patches, and, for example, reject clusters that comprise rejected (pruned away) patches and/or reduce such clusters by removing the rejected patches from that cluster. Therefore, the order of the pruning and clustering steps is not necessarily predetermined, e.g. the pruning may be executed after the clustering. However, it will also be understood that performing the pruning before the clustering (and limiting the clustering to the accepted patches as such) may be the simpler strategy, might be (typically) more efficient, may be less prone to errors and/or may be more robust.

Furthermore, both clustering steps 103, 113 may be combined, e.g. for the sake of efficiency. However, each cluster that is determined by the clustering step(s) preferably corresponds exclusively to either the first region or the second region, e.g. such that a single cluster cannot comprise both patches of the first region and of the second region simultaneously. Therefore, if the clustering steps 103 and 113 are combined (without limitation thereto), e.g. by clustering the combined set of patches (preferably after the pruning has been applied) of both regions and regardless of their assignment to either the first region or the second region, mixed clusters may be obtained as part of the clustering result, e.g. in which such a mixed cluster comprises both patches of the first and second region. Since mixed clusters are preferably to be avoided, each mixed cluster may be simply rejected (i.e. the cluster being further ignored), may be split into two clusters (one for each of the first and second region) and/or or more intricate strategy may be devised to handle this. For example, the clustering strategy may penalize mixed clusters so as to converge, e.g. by an iterative approach, to a set of clusters without such mixed clusters. Nonetheless it is noted that performing a clustering of the patches in the first region and a clustering of the patches in the second region independently from each other (preferably after pruning, in both cases) may generally be the simpler solution. Both clustering steps 103, 113 may be executed in parallel, e.g. to keep the computational burden low.

For example, after a pruning based on (e.g.) the mean pixel value of the patch, the spread of the pixel values of the patch, and/or motion, one or more (temporal) frequency characteristics of each patch may be determined (in step 109, 119). Thus, the aforementioned dynamic characteristic(s) may particularly comprise (a) temporal frequency characteristic(s), e.g. Fourier components and/or values derived therefrom.

A Fourier analysis (e.g. particularly transforming from the time domain to the temporal frequency domain) may be performed in step 109, 119, on a patch-per-patch basis. This may (optionally) be applied to (only) the patches that were not yet rejected in the pruning step, for the sake of efficiency, e.g. to reduce the burden on computational/processing resources. For example, for the patches that were not withheld by the pruning step based on mean pixel value (and/or alternative), spread (and/or alternative) and/or motion, the average pixel value (of the patch at hand) as function of time can be analysed by applying a Fourier transform, e.g. a discrete Fourier transform (DFT), e.g. a Fast Fourier Transform (FFT).

Even though, in this example, the Fourier transform is based on the mean pixel value as function of time (e.g. as the input domain for the transform), it will be understood that other suitable values to summarize the pixel content over the area of the patch can be used (as input for the FT, e.g. FFT), e.g. the central behaviour as represented by a statistical centrality measure. Alternatively, a single pixel or small group of pixels may be selected as representative for the entire patch, or even each pixel of the patch may be (Fourier-) analyzed. When multiple Fourier spectra for the same patch are calculated (e.g. for multiple pixels or multiple subgroups of pixels), the obtained frequency spectra (for the same patch) may be combined in the frequency domain, e.g. by averaging (e.g. of spectral power), even though an aggregation of data over the patch in the time domain may be preferable to the alternative of an aggregation of data in the frequency domain, e.g. for the sake of efficiency (e.g. using a Fourier transform of a single timeseries of mean pixel value, such that only one Fourier transform needs to be performed per patch).

The frequency analysis, e.g. Fourier analysis, may be performed on samples over a predetermined window of time, e.g. which is chosen appropriately, for example optimized as a parameter as discussed hereinabove, particularly so as to be sufficiently long to cover the frequencies of interest, but also sufficiently short for the sake of practicality (and/or maintain some definiteness or specificity in the time domain). The skilled person is quite familiar with the appropriate configuration of a Fourier analysis (e.g. time windows and/or sampling rates) and the considerations associated therewith. For example, the time window may be in the range of 0.5s to 30s, e.g. in the range of Is to 5s, without limitation thereto. Particularly, the Fourier analysis may be configured, and/or pre-processed and/or post-processed (e.g. using low, band and/or high pass filtering), to cover the spectral heart band, e.g. in which the PPG signal of interest is presumed to be found. For example, a band pass filter of e.g. 0.4 Hz to 4 Hz may be used, which corresponds to heartrate-synchronous signal components under typical heartrate conditions (e.g 24-240 bpm). However, embodiments are not necessarily limited thereto, e.g. suitable filter parameters may be determined by routine experimentation, simulation and/or straightforward design techniques in view of the knowledge of the person skilled in the art.

It will be understood that various alternatives to Fourier analysis can be applied, such as wavelet analysis, time-scale analysis, multi-resolution analysis and/or the like, to achieve a same or similar effect, i.e. to express the dynamic characteristic(s) of the camera signals associated with each patch. Therefore, embodiments may comprise the use of such straightforward alternative(s) instead of Fourier analysis, and/or may combine Fourier analysis with another such technique or techniques.

A frequency analysis, e.g. a Fourier spectrum, determined 109, 119 for use in clustering 103, 113 the patches based on the dynamic characteristic(s) of the patches, may also be used for a further step of pruning. This may comprise determining (per patch) the spectral energy of the strongest peak of the Fourier spectrum (and/or a predetermined band thereof, e.g. a heart rate frequency band), and/or a ratio of the spectral energy of this strongest peak over the spectral energy of the second strongest peak. This peak spectral energy, and/or this ratio, may be applied 122 as a further pruning criterium, e.g. so as to reject patches with weak or non-distinct (e.g. multiple peaks instead of a single, well-defined, strong peak) maxima, e.g. by rejecting patches for which this peak spectral energy and/or ratio is smaller than a predetermined threshold. Such further pruning 122 may be applied to both the patches in the first region and the second region, or only to the patches of interest in the first region. The further pruning criteria may be the same for the first and second region, or may differ.

For example, before continuing to the region-of-interest selection discussed hereinbelow, such further pruning 122 step may be used, e.g. as an additional validation step, to consider the correspondence of the signal strength and/or spectral property/properties (signal dynamics characteristic or characteristics) of each patch with the desired/expected properties of the PPG signal. Potential criteria may include a thresholding (one-side or two-side) of the peak spectral energy and/or of the ratio of the peak spectral energy to the average spectral energy (and/or to the second peak's spectral energy). For example, patches with weak peaks may be rejected and/or patches with a non-distinct peak (relative to the average and/or to the second largest peak) may be rejected. Furthermore, if the spectral peak is too high in amplitude, it may also be considered as an unlikely candidate for further consideration (e.g. thresholding operations may be based on not only a lower limit, but optionally also by taking an upper limit into account). Predetermined thresholds may take the predetermined patch size and predetermined length of observation window (FFT window) into account, e.g. may be easily determined by the skilled person based on the selected operational parameters.

Additionally or alternatively, such measures (e.g. peak signal strength, peak frequency, ...) may also be used in determining the dynamic characteristic(s) for further use in clustering, as discussed hereinbelow, e.g. the further pruning step (binary rejection) may be replaced or complemented by using the underlying metric(s) in the clustering step(s) discussed further hereinbelow. For example, a metric (e.g. a probability) may be associated with each patch based on the peak magnitude, based on the peak frequency, or based on a combination thereof, to build clusters from the patches in the following steps.

Thus, additionally to a further pruning, or without such further pruning, the peak frequency of the strongest peak, or the peak frequency as well as the peak magnitude, may then be used for the clustering step(s) 103, 113. The peak magnitude may optionally be expressed as a ratio relative to the second largest peak, to the average over the spectral band under consideration (heart rate band), and/or another suitable expression. The frequency and magnitude may be combined in a weighted scalar combination, e.g. combining a fitness (e.g. a probability) of the frequency and a fitness of the magnitude into a combined fitness (probability, likelihood, ...) for clustering the patches, or may be used together as a vector value for the clustering.

The clustering 103, 113 may be based on (e.g. at least) the frequency of the strongest spectral peak, as determined per patch (e.g. for patches not withheld by the initial step of pruning and/or the further step of pruning). However, alternatives can be used as well, e.g. determining the time scale in which the strongest signal is detected (e.g. highest power or a measure of intensity, magnitude, ...), e.g. using a multi-resolution time-scale analysis (e.g. using a hierarchical pyramid of scale analysis) and associating a scale value of this (most prominent) time resolution with the patch for use as the clustering variable. Clustering based on multiple variables, e.g. associating vector-valued variables with the patches to use as basis for the clustering, is not necessarily excluded either, e.g. combining a peak frequency and a peak time scale.

For example, the peak frequency (or a suitable equivalent, e.g. a scale in a scale hierarchy with highest observed energy, a time period corresponding to the peak frequency, a wavelength, ...) may be used to determine clusters of patches in which pixels are pulsating at substantially the same or similar frequency (with respect to the other patches in the cluster). As mentioned, a qualifier of the signal strength (for this frequency component) may be included in the clustering variable as well.

By using the same, or similar, clustering variable (e.g. peak frequency and/or magnitude) for clustering the patches of the first region as well as clustering the patches of the second region, information from the non-relevant clusters in the second region can be used to select one or more clusters of relevant patches in the first region, as discussed hereinbelow.

The method comprises selecting 104 at least one cluster from the clusters of patches obtained by the step of clustering 103 the patches of the first region based on size and/or morphology. Then, an output is provided 130. A region of interest (ROI) definition may be provided as output, which defines the pixels that form the selected cluster or clusters, e.g. for use by a dedicated PPG signal extraction algorithm, and/or a PPG signal may be determined 105 from the pixel values in said selected cluster(s) (in a further sequence of images acquired by the camera). For example, the PPG signal may be determined 105 based on dynamic changes in (e.g. the spatial average over) substantially only the pixels of the selected cluster(s). In other words, the selected cluster(s) can be used to calculate a robust PPG signal (from the pixels corresponding to the ROI in a sequence of camera images), either directly by a further step 105 of the method in accordance with embodiments of the present invention or by providing the ROI to an external algorithm, as known in the art, to determine the PPG signal from a sequence of the camera images based on the received input that represents (or comprises) the ROI definition.

However, the step of selecting 104 the cluster(s) comprises excluding those clusters found for the first region that correspond to a cluster found by clustering 113 the patches of the second region. Particularly, the step of selecting 104 the cluster(s) excludes from said selection each cluster of the first plurality of subsets that substantially corresponds in its associated temporal signal dynamics to the temporal signal dynamics of a cluster of the second plurality of subsets, e.g. for which the (absolute) difference (or any suitable distance metric) between the at least one dynamic characteristic values of the pair of clusters under evaluation, one from the first region and the other from the second region, is below a predetermined threshold. For example, for each cluster of the first region, the distance (in general, e.g. a metric based on the dynamic characteristics) to each cluster of the second region may be determined, and this cluster of the first region may be rejected if the minimum distance thus found is below the threshold. Optionally, only the largest clusters of the second region may be taken into account for this exclusion step, e.g. so as to avoid rejecting a large cluster of the first region that matches a small cluster, e.g. a stray patch or a few stray patches of the tissue of interest (e.g. skin), in the second region. In other words, a size criterion may be applied to the clusters of the second region so as to avoid filtering out clusters of the first region based on a match to a small cluster of the second region that could correspond to a small area of the tissue of interest that was incorrectly presumed to be irrelevant (by the a-priori assignment of the corresponding image area to the second region).

To select 104 the cluster(s) based on size, the largest cluster, or a predetermined number of largest clusters, may be selected from the clusters found by clustering 103 the patches of the first region, after said exclusion of corresponding clusters. The size of each cluster, for selecting the largest cluster(s), may be determined based on the number of patches, the number of pixels, the corresponding area, or another similar measure expressing the spatial extent of the cluster. Thus, a cluster is selected which comprises an advantageously large (in so far possible) number of pixels, but does not match dynamic characteristics encountered for the second region. The pixels belonging to the selected cluster or clusters can then be used to define a region of interest from which a robust and accurate PPG signal can be extracted.

Instead of directly selecting the largest cluster (of patches found for the first, relevant, region), the largest cluster is selected that is not associated with any of the irrelevant (e.g. non-skin) frequencies (or an alternative dynamic characteristic used for the clustering); i.e. clusters corresponding to oscillations and/or dynamics of the pixel signal (e.g. pixel intensity) that are associated with non-skin (or, more generally, not to the tissue type of interest) are ignored, i.e. excluded from the selection.

For example, the step of selecting 104 a cluster from the clusters of patches obtained by clustering 103 the patches of the first region may comprise pruning clusters from these clusters of patches of the first region that correspond in their dynamic characteristic value(s), e.g. peak frequency, to the dynamic characteristic value(s), e.g. peak frequency, of a cluster obtained by clustering 113 the patches of the second region.

In an illustrative approach, the peak frequencies (or alternative therefor) are averaged over patches in each cluster to associate a single peak frequency to the cluster, and cluster peak frequencies (or alternative) that are found for both the first region and the second region may be removed (i.e. pruning away the corresponding clusters in the first region, or simply ignoring such clusters). The peak frequencies may be compared (between clusters in respectively the first and second region) by simply thresholding the absolute difference thereof (e.g. requiring the difference to be less than a predetermined threshold to match). Alternatives can be easily envisioned. For example, the standard deviation of the peak frequency in a cluster (over the patches forming the cluster) may be taken into account, e.g. comparing two clusters by means of a Student's t-test, F-test or the like. Optionally, small clusters in the second region may be ignored, e.g. a criterion may be imposed that the matching cluster frequency corresponds to a cluster of at least a substantial size in the second region (e.g. a predetermined size threshold). Thus, in order to be rejected from the selection, a (large) cluster in the first region may be required to match in frequency to a cluster in the second region that has at least a substantial size as well. After removing the clusters of the first region with corresponding clusters in the second region (with respect to the dynamic parameter used for clustering, e.g. peak frequency), the largest, or top ranking set of largest (e.g. top 2, top 3, ...), clusters of the first region that is still under consideration may be selected to determine the region of interest, i.e. to be used for extraction of the PPG signal.

Whereas, for the sake of simplicity and efficiency, corresponding clusters in the first region and the second region are described as being determined based on the dynamic characteristic used for clustering (e.g. peak frequency), it will be understood that this is not strictly necessary. The same or similar effect can also (additionally or alternatively) be obtained by comparing the temporal dynamics of clusters in the first and second regions by another suitable measure, e.g. using a cross-correlation of the average signal (over the spatial extent in each of the pair of clusters being compared; or of an alternative value of centrality or the like) over time, using a statistical test to compare the distributions, using mutual information or another information-theoretic metric, and/or using another suitable measure to compare a scalar or vector representative of each of the pair of clusters under comparison, e.g. a measure for comparing time series, Fourier spectra, and/or other qualities of dynamic behavior.

Additionally, the selection 104 may also take morphology of each cluster into account. Particularly, each cluster, found in the first region of (presumed relevance as) the tissue of interest, e.g. skin, may be associated with a measure based on the shape of the image area formed by the pixels (or patches) in the cluster (i.e. of the ensemble of patches in the cluster, and/or of the combined set of pixels forming the cluster), e.g. so as to rate clusters for consistency with the expected shape of an area of the tissue of interest. Thus, for each cluster, not rejected by a correspondence with a matching cluster of irrelevant image content (i.e. in the second region), a measure, e.g. a probability index, may be determined 121 that indicates if (and/or to which extent) the shape of the image area formed by the pixels (or patches) in the cluster is consistent with that of the expected shape.

For example, the forehead of the subject may form a target region of tissue of interest (skin), which would correspond to a connected set of patches in an approximately oval shape. Thus, in this example, the measure that may be determined 121 for each cluster (of the first region; e.g. not rejected by a previously applied pruning step) may express the correspondence of the cluster to an oval shape. Equivalently, the measure may express a deviation therefrom, i.e. the measure may be a substantially monotonous relationship that increases, or equivalently decreases, when the shape of the image area formed by the pixels (or patches) in the cluster matches the intended shape better.

For example, an oval or ellipse may be fitted to the patches (or, equivalently, the set of pixels) forming the cluster, and a (shape) error value may be assigned for patches (resp. pixels) extending beyond the fitted shape and to missing patches inside the interior of the shape. Thus, if an ensemble of patches/pixels (i.e. the cluster) forms (e.g.) a line segment, it is unlikely to detail the desired feature, e.g. a face or part thereof, e.g. the forehead. Furthermore, alternatively or additionally, the measure (e.g. an error value) may take the fitted parameters, e.g. the major and minor axis (and/or ratio thereof) of the fitted ellipse, into account, e.g. so as to prefer fitted ellipse (or oval) regions that are in a range corresponding to the expected overall shape to be found. For example, a fitted ellipse that has a very large ratio of the largest to smallest axis would be more consistent with a line segment than with a desired target shape (in this example) of an ellipse closer to a (roughly) circular form.

Many variations can be easily envisioned, based on the expected shape of the target region of interest. For example, for targeting a section of skin on the forearm, an approximately rectangular area may be used as criterion for defining the shape measure, e.g. by fitting a rectangle or rounded rectangle to the cluster (e.g. at an arbitrary angle with respect to the camera image coordinates). Thus, in this example, the selection may actively prefer more elongate clusters based on prior knowledge (e.g. the intended use for extracting the PPG signal from camera observation of the forearm).

A cluster may thus be selected based on size and/or morphological probability, e.g. a combination of both, while rejecting corresponding clusters (based on the dynamics, e.g. the peak Fourier frequency) in the second region (presumed to be irrelevant for the tissue of interest). Therefore, embodiments of the present invention may improve upon a prior-art "living skin" selection method by taking information into account that is obtained in (an) area(s) of the image where the tissue of interest is unlikely to be found (i.e. the signal dynamics in the second region discussed hereinabove), and, optionally, also by taking morphological properties of the selected cluster into account (e.g. a shape criterion).

The different contributions to the selection (e.g. ranking) step, for example elements based on a correspondence with a non-relevant cluster in the second region, based on the cluster size and based on the cluster's shape, may be combined in various ways. For example, the different types of measures may be weighted and combined to obtain a ranking measure to select the most promising cluster or clusters. Alternatively, a binary exclusion from the selection may be applied for one or more of the measures, e.g. rejecting clusters with corresponding dynamic properties in a cluster of the second region and/or rejecting clusters that are too small or too large and/or rejecting clusters that do not correspond to the desired shape. A combination thereof may also be used, e.g. using some of these measures for binary selection and one or more for a selection by ranking after applying the binary selection. The measures used for binary selection and ranking selection may be different, or may overlap. For example, a first rejection step may be based on these criteria with a relatively tolerant threshold, and a weighted combination of these measures may be used for a final selection by ranking.

In a second aspect, the present invention relates to a device for use in extracting a photoplethysmography signal indicative of a subject's physiology based on remote camera observation, for example to provide such PPG signal and/or to provide a region-of-interest that is adapted to select pixels in the image frames provided by the camera to extract such PPG from.

Referring to FIG. 2, an illustrative device 10 in accordance with embodiments of the present invention is shown schematically.

The device 10 comprises an input 11 for receiving camera images from a camera configured to monitor at least one body part of the subject. The device 10 may optionally comprise the camera 19, or the device may be adapted to connect to such (external) camera, e.g. via a wired, wireless or indirect (e.g. retrieving images stored on an intermediate device, e.g. a streaming server) connection. The device may optionally also comprise a light source (or sources) to illuminate the (relevant body part or parts of the) subject. The camera may comprise an infrared camera, a monochrome camera operating in the visible wavelength range or part thereof, e.g. a green light spectral band, a color camera, and/or a multi-spectral camera. Likewise, the light source may emit light in at least an overlapping part of the spectrum to which the camera is sensitive, e.g. an infrared light source, a green light source, a broadspectrum (e.g. white) light source, ...

The device 10 comprises a subset generator 12 for dividing a first region, representative of an image part in the camera images where a tissue of interest is presumed to be present, into a first plurality of subsets of pixels (e.g. patches) and dividing a second region, different from the first region and representative of an image part in the camera images where the tissue of interest is presumed to be absent, into a second plurality of subsets of pixels (e.g. patches).

The subset generator 12 may also be adapted to divide the first region, and respectively the second region, into the first, respectively the second, plurality of pixel subsets based on predetermined prior-knowledge assumptions of where the tissue of interest is likely present, respectively absent, given a known setup of the camera with respect to a volume in space where the subject is to be positioned. Such prior-knowledge may, for example (without limitation thereto), be hardcoded or hardwired in the device, may be stored in a suitable configuration memory and/or may be received via a user interface or interface to an external controller.

The subset generator 12 may be adapted to divide the first region, respectively the second region, into the first, respectively second, plurality of pixel subsets by forming a partition of the first region, respectively of the second region, into blocks or patches based on a spatial distance metric, such that each subset groups pixels together that are close in space. Additionally or alternatively, the respective regions may be partitioned into subsets based on an image codomain distance, e.g. grouping pixels that are close to each other in their pixel value. A combination of spatial and codomain based distance may also be used, e.g. such that and/or based on a combination thereof, such that each subset groups pixels together that are close in (a balanced combination of) space and in value.

The subset generator 12 may be adapted to perform an image segmentation and/or processing of a reference image (and/or, in general, reference spatial information) received via the input 11, e.g. from the camera, from a further camera and/or from a (generic) spatial information source (e.g. a 3D surface scanner, a tomographic imaging system, ...), to determine the first and second regions.

The subset generator 12 may be adapted to perform an image subtraction between a reference image acquired with the subject present and a blank image acquired without the subject present.

The device may also comprise a subset eliminator 18 for pruning the first plurality of subsets based on predetermined criteria for one or more values determined on a subset-per-subset basis, so as to reject subsets that have a low likelihood of corresponding to homogeneous areas of the tissue of interest. The subset eliminator 18 may also be adapted to prune the second plurality of subsets based on the same and/or further predetermined criteria. The clustering processor 14, discussed further hereinbelow, may be adapted to cluster respectively the pruned first plurality of subsets and the pruned second plurality of subsets, e.g. as determined by the subset eliminator.

The subset eliminator 18 may be adapted to prune the first and/or second plurality of subsets based on predetermined criteria comprising one or more criteria for: a mean pixel value per subset, a spread of the pixel values per subset and/or at least one value indicative of motion associated with the subset, such that the subset eliminator is adapted to reject subjects of pixels that show excessive motion, an inhomogeneous pixel value distribution and/or a pixel value that is, on average, outside a predetermined target range. It will be understood that various alternatives and/or equivalents can be used for a mean (e.g., generally, a measure of centrality), a spread (e.g., generally, a measure of dispersion, e.g. a variation or standard deviation) and motion (e.g. motion vector components, a motion vector magnitude, a squared motion vector magnitude, ...).

The device comprises a signal dynamics analyzer 13 for determining, per subset of the first plurality of subsets and per subset of the second plurality of subsets, at least one dynamic characteristic value that is indicative of temporal signal dynamics of the subset in a sequence of the camera images received via the input 11.

The signal dynamics analyzer 13 may be adapted to perform a Fourier transform in the temporal domain to obtain one or more temporal frequency characteristics for use in determining the at least one dynamic characteristic value.

The subset eliminator 18 may be adapted to apply a further pruning of the (e.g. already a first time pruned, cf. hereinabove) first and/or second plurality of subsets based on the at least one dynamic characteristic value. Thus, the clustering processor 14 may be adapted to cluster respectively the further pruned first plurality of subsets and the further pruned second plurality of subsets.

For example, the further pruning may be based on at least one predetermined criterion for the spectral energy of the largest spectral peak of the Fourier spectrum determined for each subset and/or based on a value determined therefrom, e.g. rejecting subsets for which this spectral energy is below a certain threshold or outside a predetermined value range.

The device comprises a clustering processor 14 for clustering, respectively, the first plurality of subsets and the second plurality of subsets, based on the at least one dynamic characteristic value, so as to group subsets of pixels into clusters with similar signal dynamics.

The device comprises a selector 15 for selecting at least one cluster from the clusters of the first plurality of subsets provided by the clustering processor 14 based on cluster size and/or cluster morphology. The selector is adapted to exclude, from the selection, each cluster of the first plurality of subsets that substantially corresponds in its associated signal dynamics to the signal dynamics of a cluster of the second plurality of subsets.

Referring to said cluster size based selection, the selector 15 may be adapted to select the largest cluster or the largest clusters from the first plurality of subsets that is, or are, not excluded based on their correspondence to a cluster of the second plurality of subsets.

For a cluster morphology based selection, the selector may be adapted to determine, for each cluster of the first plurality of subsets (or for a preliminary selection from those clusters, e.g. after applying a rough first selection criterion, e.g. eliminating clusters that are too small, and/or after the exclusion based on correspondence to a cluster of the second plurality of subsets), a shape measure representative of the shape of the image area formed by the pixels (or patches) in the cluster, and to select the at least one cluster (i.e. the result to be used in the ouput) based on the shape measure of the selected cluster(s) being consistent with a predetermined target shape of an area of the tissue of interest.

The selector 15 may also be adapted to determine a ranking of the clusters of the first plurality of subsets that are not excluded based on said correspondence to a cluster of the second plurality of subsets, in which this ranking is used to determine the selected cluster(s). The ranking may combine a first score based on a size of the cluster and a second score based on the shape measure, e.g. so as to combine a selection based on morphology and on size. It will be understood that the exclusion criterion (correspondence to a cluster of the second plurality of subsets) may also be introduced into this ranking, e.g. such that the exclusion is imposed by a penalty to the ranking. In other words, the exclusion may be a hard exclusion that is executed preemptively, or may be imposed by a soft requirement, e.g. such as to reduce the ranking of clusters that match in their dynamic characteristics to a cluster of the presumed irrelevant second region. It is also noted that this may avoid or reduce the limitations due to a strictly imposed criterion on a match between clusters of the first and second plurality of subsets. While this may be implemented by, for example, requiring an absolute difference between the peak frequency to be smaller than a predetermined threshold, by alternatively including this absolute difference (or a similar measure or equivalent) into the ranking measure, e.g. appropriately weighted, a particularly large cluster with the expected shape can be selected even if it is relatively close (but not "too close") in its dynamic characteristics to a cluster in the second set.

The device comprises an output 16 to output a region-of-interest definition that defines the pixels forming the at least one cluster selected by the selector 15 for use in extracting a photoplethysmography signal from camera images acquired by the camera, or, alternatively or additionally, to output a photoplethysmography signal extracted from the region-of-interest in camera images received via the input 11.

Therefore, the device may optionally also comprise a photoplethysmography signal extractor 21 to extract, from the region-of-interest in (a sequence of) camera images, received via the input 11 from the camera, the photoplethysmography signal to be provided as output.

The device may also comprise an optimizer 17. The subset generator 12 may be adapted to repeatedly divide the first region into a first plurality of pixel subsets (and optionally, also the second region into a second plurality of subsets) for different values of at least one optimization parameter representative of the size of the pixel subsets. The optimizer 17 may be adapted for calculating and evaluating a quality metric based on the pixel subsets obtained in each such iteration for a different value of the at least one optimization parameter, such that the optimizer can select the parameter value for which a sufficient (e.g. above a predetermined threshold, or after a predetermined number of iteration steps, or up to a predetermined parameter accuracy, ...) or optimal value of the quality metric Is obtained. The thus selected parameter value(s) and the corresponding division of the first and second region into subsets may then be used by the clustering processor 14 and selector 15, e.g. may be used to determine the region-of-interest.

The optimizer 17 may also use, as a component of the at least one optimization parameter (e.g. in addition to the subset size being optimized), a size of a temporal observation window used by the signal dynamics analyzer 13, e.g. a Fourier time window parameter.

The optimizer 17 may, for example, calculate a quality metric that comprises or consists of a signal-to-noise ratio.

For example, the device may comprise a processor, computer, or similar general-purpose computing device, in combination with software adapted to perform a method in accordance with embodiments of the present invention, as discussed hereinabove. The device may alternatively or additionally comprise dedicated hardware designed for performing the method, or (a) step(s) thereof, in accordance with embodiments of the present invention. For example, such dedicated hardware may comprise an application-specific integrated circuit, and/or configurable hardware, such as a fieldprogrammable gate array.

In a third aspect, the present invention relates to a diagnostic imaging system, such as a magnetic resonance imaging system or a computed tomography system. The system has an examination zone and comprising a camera for monitoring a subject when undergoing an examination while positioned in the examination zone. The system also comprise a device in accordance with embodiments of the second aspect of the present invention, operably connected to the camera so as to receive camera images from the camera as input, e.g. from a camera 19 via the input 11.

As known in the art, the system may be adapted for imaging the subject by magnetic resonance imaging, computed tomography, positron emission tomography and/or single-photon emission computed tomography. Alternatively, in a further aspect, the present invention relates to similar system for performing a medical intervention, such as a radiotherapy system, e.g. comprising a camera system and device in accordance with embodiments of the second aspect of the present invention. Other embodiments may include a system for monitoring a patient during a surgical intervention, in an intensive care unit and/or, generally, for monitoring patient health in a medical or paramedical setting.

For example, the system may be a magnetic resonance imaging system, however principles of the present invention can equally be applied to a system for a different diagnostic imaging modality and/or for use in another medical or nonmedical context.

A magnetic resonance examination system in accordance with embodiments may comprise a primary magnet assembly, which defines the examination zone, e.g. the examination zone may be formed by a volume where the magnetic field conditions, as substantially created and controlled by the magnet assembly, are suitable for magnetic resonance imaging. The examination zone may thus correspond to (at least a usable portion of) the volume enclosed by a magnet bore of the system (without limitation, e.g. principles of the present invention equally apply to open bore systems and other, less frequently used, magnet assembly configurations).

A subject, e.g. a patient, to be examined may, in use of the system, be positioned on a patient couch in the examination zone. The primary magnet assembly may comprise magnet windings, e.g. coaxial (e.g. superconductive) windings, to generate a stationary uniform magnetic field in the examination zone. The examination zone may be a cylindrical volume encompassed by these magnet windings.

The system may comprise a reconstructor to reconstruct magnetic resonance image(s), e.g. tomographic MRI images, from magnetic resonance signals acquired by the system in use. The reconstructed images may be provided via an output for viewing, processing or storage.

Auxiliary equipment, such as an RF T/R head coil may, in use, be place in the examination zone to acquire magnetic resonance signals from the subject's head. Other auxiliary coil configurations may be used to acquire signals from other body parts or for different use cases, while, typically, signals may also be received by receiver coils already integrated in the housing of the primary magnet assembly.

The system comprises a camera, or camera assembly, e.g. comprising multiple cameras. The camera system is adapted to obtain information from the subject being examined, e.g. to obtain vital signs, motion, indicators of distress and the like.

For example, the camera may be mounted close to one entry of the examination zone. For example, the camera may be integrated in, or mounted on, a flange of the MR bore (e.g. such that the usable free bore diameter is not affected or only minimally reduced, and/or to avoid or minimize interference with the operation of the MR system). For example, (optional) illuminating lights may also be provided in or on this flange (without limitation thereto).

The system may comprise a display to display images of the inside of the examination zone acquired by the camera (raw, and/or after suitable processing). This enables an operator to visually monitor the subject in the examination zone.

The images acquired by the camera may be provided to a device in accordance with embodiments, e.g. to an image processor for executing a method in accordance with embodiments. Thus, the image processor may be or may comprise a device 10 in accordance with embodiments of the second aspect of the present invention. The image processor (i.e. the device 10) is adapted to process image information acquired by the camera system, and to perform image analysis to obtain information from the patient, particularly to extract a photoplethysmography (PPG) signal from a region-of-interest in the video stream acquired by the camera, in which this ROI is obtained as detailed hereinabove.

Respiratory and/or cardiac phase information (and/or more generic information indicative of motion), e.g. the PPG signal generated by the device and/or information derived therefrom and/or enriched thereby, may be provided to the reconstructor to correct the acquired magnetic resonance signals for motion and/or apply motion corrections to the reconstructed magnetic resonance images. For example, a cardiac trigger signal may be determined based on the PPG signal.

Thus, the signal provided by the device 10 may be used to gate a data acquisition by the system, e.g. an MRI system or other system for performing a diagnostic imaging procedure. Or additionally, or alternatively, to control the delivery of a therapeutical procedure using a therapeutical system. Additionally or alternatively, the signal may be used to sort, collate, select and/or annotate acquired image data by the diagnostic imaging system, e.g. to show extracted PPG information (or information derived therefrom, such as a cardiac phase) alongside the diagnostic image(s) acquired at substantially the corresponding time.

In a magnetic resonance imaging system in accordance with embodiments of the present invention, the camera system may also comprise one or more light sources. While embodiments that rely on passive lighting for imaging are not necessarily excluded, it will be understood by the skilled person that lighting conditions can be better controlled, and (camera) imaging can be more effective, when using active lighting.

The light source and/or the camera may be located outside the examination zone, or on or near an edge region thereof. This can simplify the configuration of the magnetic resonance imaging system (e.g. avoiding or reducing interference with the RF and magnetic field operation of the system) and may provide for more free bore width in the examination zone. For example, for a cylindrical bore system, both camera and light source (or either one thereof individually) may be located at the flange of the bore enclosure at one end of the bore, which may leave the other end substantially free, e.g. to allow unimpeded access to the examination zone (for bringing the patient and/or auxiliary equipment into the examination zone), and reducing a potential claustrophobic effect on the subject, and thus possible discomfort, while being imaged by the system.

In a magnetic resonance imaging system in accordance with embodiments of the present invention, the camera (or cameras) may be adapted to operate (e.g. substantially exclusively sensitive to) light in a (e.g. narrow) infrared wavelength range and outside the visible wavelength range.

In a magnetic resonance imaging system in accordance with embodiments of the present invention, the camera (or cameras) may be adapted to operate (e.g. substantially exclusively sensitive to) in the visible wavelength range, e.g. sensitive to a broad white light spectrum, or part thereof, e.g. a color band, e.g. green light. The camera may be adapted to acquire monochrome information, or may be a color camera, e.g. adapted to detect, preferably independently and substantially simultaneously, different color components, e.g. a red, green and blue component (without limitation thereto). The camera may also be adapted to detect a relatively large (e.g. more than three) spectral components, e.g. may be a multispectral camera.

The light source(s) may emit light in a spectrum suitable for the camera, e.g. a broadband white light may provide illumination for a monochrome or color camera in the visible range to operate. Likewise, an infrared light source may be used to emit infrared light in a spectral range in which the infrared camera is sensitive. It will be understood that the spectra of the light source and the camera are not necessarily identical or not even necessarily closely related, e.g. the spectrum of the light source may be broader in so far that sufficient overlap exists with the spectrum to which the camera is sensitive. The camera may be a digital camera, e.g. comprising an array of pixel light detectors.

In a fourth aspect, the present invention relates to a computer -program product for performing a method in accordance with embodiments of the first aspect of the present invention when executed by a computing device. For example, the computer-program product may comprise machineinterpretable instructions to direct the computing device, e.g. a computer, to implement (i.e. execute) the method of embodiments.

Other features, or details of the features described hereinabove, of a device, system and/or computer-program product in accordance with embodiments of the present invention shall be clear in view of the description provided hereinabove relating to a method in accordance with embodiments of the present invention, and/or vice versa, i.e. the details and features discussed hereinabove, regardless of their context (the embodiment and/or aspect of the invention being described), may apply equally to all the different aspects of the present invention, mutatis mutandis.

## Claims

1. A device (10) for use in extracting a photoplethysmography signal indicative of a subject's physiology based on remote camera observation, the device comprising:
- an input (11) for receiving camera images from a camera (19) configured to monitor at least one body part of the subject;
- a subset generator (12) for dividing a first region, representative of an image part in said camera images where a tissue of interest is presumed to be present, into a first plurality of subsets of pixels and dividing a second region, different from the first region and representative of an image part in said camera images where the tissue of interest is presumed to be absent, into a second plurality of subsets of pixels;
- a signal dynamics analyzer (13) for determining, per subset of the first plurality of subsets and per subset of the second plurality of subsets, at least one dynamic characteristic value that is indicative of temporal signal dynamics of the subset in a sequence of the camera images received via the input (11);
- a clustering processor (14) for clustering, respectively, the first plurality of subsets and the second plurality of subsets, based on the at least one dynamic characteristic value, so as to group subsets of pixels into clusters with similar signal dynamics;
- a selector (15) for selecting at least one cluster from the clusters of the first plurality of subsets provided by the clustering processor (14) based on cluster size and/or cluster morphology, in which the selector is adapted to exclude from said selection each cluster of the first plurality of subsets that substantially corresponds in its associated signal dynamics to the signal dynamics of a cluster of the second plurality of subsets, and
- an output (16) to output a region-of-interest definition that defines the pixels forming the at least one cluster selected by the selector (15) for use in extracting a photoplethysmography signal from camera images acquired by the camera and/or to output a photoplethysmography signal extracted from the region-of-interest in camera images received via the input (11).

2. The device of claim 1, comprising a photoplethysmography signal extractor (21) to extract, from said region-of-interest in a sequence of said camera images received via the input (11), the photoplethysmography signal to be provided via the output (16).

3. The device of any of the previous claims, wherein said selector (15) is adapted to select the largest cluster or the largest clusters from the first plurality of subsets that is, or are, not excluded based on said correspondence to a cluster of the second plurality of subsets, and/or
wherein said selector (15) is adapted to determine for each cluster of the first plurality of subsets a shape measure representative of the shape of the image area formed by the pixels and/or patches in the cluster, and to selecting said at least one cluster based on the shape measure of the selected cluster being consistent with a predetermined target shape of an area of the tissue of interest, and/or
wherein said selector (15) is adapted to determine a ranking of the clusters of the first plurality of subsets, in which said ranking is used to determine said selection and said ranking combines a first score based on a size of the cluster and a second score based on the shape measure.

4. The device of any of the previous claims, wherein said subset generator (12) is adapted to divide the first region, respectively the second region, into said first, respectively second, plurality of pixel subsets by forming a partition of the first region, respectively the second region, into blocks or patches based on a spatial distance metric and/or into subsets based on an image codomain distance and/or based on a combination thereof, such that each subset groups pixels together that are close in space and/or in value.

5. The device of any of the previous claims, wherein said subset generator (12) is adapted to perform an image segmentation and/or processing of a reference image and/or reference spatial information received via the input (11) to determine said first and second regions, and/or to perform an image subtraction between a reference image acquired with the subject present and a blank image acquired without the subject present, in which the reference image and/or reference spatial information is received via the input (11) as acquired by the camera, a further camera and/or a further spatial information source.

6. The device of any of the previous claims, wherein said device comprises an optimizer (17), and said subset generator (12) is adapted to repeatedly divide the first region into said first plurality of pixel subsets for different values of at least one optimization parameter representative of the size of the pixel subsets, the optimizer (17) being adapted to calculate and evaluate a quality metric based on the pixel subsets obtained in each iteration for a different value of the at least one optimization parameter so as to select the parameter value for which a sufficient or optimal value of the quality metric Is obtained.

7. The device of any of the previous claims, wherein said optimizer (17) also uses, as component of said at least one optimization parameter, a size of a temporal observation window used by said signal dynamics analyzer (13), and/or wherein said said quality metric calculated by said optimizer (17) comprises or consists of a signal-to-noise ratio.

8. The device of any of the previous claims, wherein said signal dynamics analyzer (13) is adapted to perform a Fourier transform in the temporal domain to obtain one or more temporal frequency characteristics for use in determining said at least one dynamic characteristic value.

9. The device of any of the previous claims, comprising a subset eliminator (18) for pruning the first plurality of subsets based on predetermined criteria for one or more values determined on a subset-per-subset basis, so as to reject subsets that have a low likelihood of corresponding to homogeneous areas of the tissue of interest, and for pruning the second plurality of subsets based on the same and/or further predetermined criteria, and wherein said clustering processor (14) is adapted to cluster respectively the pruned first plurality of subsets and the pruned second plurality of subsets.

10. The device of claim 9, wherein said subset eliminator (18) is adapted to prune said first and/or second plurality of subsets based on the predetermined criteria comprising one or more criteria for: a mean pixel value per subset, a spread of the pixel values per subset and/or at least one value indicative of motion associated with the subset, such that the subset eliminator is adapted to reject subjects of pixels that show excessive motion, an inhomogeneous pixel value distribution and/or a pixel value that is, on average, outside a predetermined target range.

11. The device of claim 9 or 10, wherein said subset eliminator (18) is furthermore adapted to apply a further pruning of the first and/or second plurality of subsets based on the at least one dynamic characteristic value, and wherein said clustering processor (14) is adapted to cluster respectively the further pruned first plurality of subsets and the further pruned second plurality of subsets.

12. A method (100) for use in extracting a photoplethysmography signal indicative of a subject's physiology based on remote camera observation, the method comprising:
- acquiring (115) camera images from a camera configured to monitor at least one body part of the subject;
- dividing (101) a first region, representative of an image part in said camera images where a tissue of interest is presumed to be present, into a first plurality of subsets of pixels and dividing (111) a second region, different from the first region and representative of an image part in said camera images where the tissue of interest is presumed to be absent, into a second plurality of subsets of pixels;
- determining (109,119), per subset of the first plurality of subsets and the second plurality of subsets, at least one dynamic characteristic value that is indicative of temporal signal dynamics of the subset in a sequence of the camera images;
- clustering (103,113), respectively, the first plurality of subsets and the second plurality of subsets, based on the at least one dynamic characteristic value, so as to group subsets of pixels into clusters of similar signal dynamics;
- selecting (104) at least one cluster from the clusters of subsets obtained by the step of clustering (103) the first plurality of subsets based on cluster size and/or cluster morphology, in which the step of selecting (104) the at least one cluster excludes from said selection each cluster of the first plurality of subsets that substantially corresponds in its associated signal dynamics to the signal dynamics of a cluster of the second plurality of subsets; and
- providing (130) an output, in which the output comprises a region-of-interest definition to define the pixels forming the selected at least one cluster for use in extracting a photoplethysmography signal from camera images acquired by the camera and/or comprises a photoplethysmography signal extracted (105) from the region-of-interest in the camera images acquired by the camera.

13. The method of claim 12, wherein said first region, respectively second region, is divided (101,111) into said first, respectively second, plurality of pixel subsets based on predetermined prior-knowledge assumptions of where the tissue of interest is likely present, respectively absent, given a known setup of the camera with respect to a volume in space where the subject is to be positioned.

14. A diagnostic imaging system with an examination zone (11), the system comprising:
- a camera (19) for acquiring images from a subject when undergoing an examination while positioned in the examination zone,
- the device of any of the claims 1 to 11, operably connected to the camera to receive camera images from the camera (19) as input.

15. A computer-program product for performing a method in accordance with any of the claims 12 to 13 when executed by a computing device.
